# EUROPÄISCHE PATENTSCHRIFT

(11) **EP 1 509 246 B1**
(45) Veröffentlichungstag und Bekanntmachung des Hinweises auf die Patenterteilung: **27.05.2009**
(21) Anmeldenummer: 03735485.9
(22) Anmeldetag: 28.05.2003
(51) Int. Cl.: A61K 41/00, A61K 47/48

(54) **THERMOSENSITIVE POLYMERTRÄGER MIT VERÄNDERBARER PHYSIKALISCHER STRUKTUR FÜR DIE BIOCHEMISCHE ANALYTIK, DIAGNOSTIK UND THERAPIE**
THERMOSENSITIVE POLYMER CARRIERS HAVING A MODIFIABLE PHYSICAL STRUCTURE FOR BIOCHEMICAL ANALYSIS, DIAGNOSIS, AND THERAPY
SUPPORT POLYMERE THERMOSENSIBLE A STRUCTURE PHYSIQUE POUVANT ETRE MODIFIEE, POUR L'ANALYSE BIOCHIMIQUE, LE DIAGNOSTIC ET LA THERAPIE

(30) Priorität: 01.06.2002 DE 10224352
(43) Veröffentlichungstag der Anmeldung: 02.03.2005
(73) Patentinhaber: MagnaMedics GmbH, 52066 Aachen (DE)
(72) Erfinder: MÜLLER-SCHULTE, Detlef P., 52066 Aachen (DE)
(74) Vertreter: Mann, Volker
(86) Internationale Anmeldenummer: PCT/EP2003/005614
(87) Internationale Veröffentlichungsnummer: WO 2003/101486

(56) Entgegenhaltungen:
- WO-A-01/05586
- WO-A-03/026618
- KONDO A ET AL: "DEVELOPMENT AND APPLICATION OF THERMO-SENSITIVE MAGNETIC IMMUNOMICROSPHERES FOR ANTIBODY PURIFICATION" APPLIED MICROBIOLOGY AND BIOTECHNOLOGY, SPRINGER VERLAG, BERLIN, DE, Bd. 41, 1994, Seiten 99-105, XP000613881 ISSN: 0175-7598
- KONDO A ET AL: "PREPARATION OF THERMO-SENSITIVE MAGNETIC HYDROGEL MICROPHERES AND APPLICATION TO ENZYME IMMOBILIZATION" JOURNAL OF FERMENTATION AND BIOENGINEERING, SOCIETY OF FERMENTATION TECHNOLOGY, JP, Bd. 84, Nr. 4, 1997, Seiten 337-341, XP001055080 ISSN: 0922-338X in der Anmeldung erwähnt
- CHEN J P ET AL: "Latex particles with thermo-flocculation and magnetic properties for immobilization of alpha-chymotrypsin." BIOTECHNOLOGY PROGRESS. 2001 MAR-APR, Bd. 17, Nr. 2, März 2001 (2001-03), Seiten 369-375, XP002257378 ISSN: 8756-7938
- MOSELHY J ET AL: "In vitro studies of the interaction of poly(NIPAm/MAA) nanoparticles with proteins and cells." JOURNAL OF BIOMATERIALS SCIENCE. POLYMER EDITION. 2000, Bd. 11, Nr. 2, 2000, Seiten 123-147, XP0008022878 ISSN: 0920-5063

## Beschreibung

Die Erfindung betrifft thermosensitive Polymere, die aufgrund eingekapselter magnetischer und/oder metallischer Kolloide mittels magnetischer Induktion erwärmt werden können und dadurch eine Änderung der physikalischen Struktur oder Form erfahren. Die mit der Erwärmung einhergehende Formveränderung wird u.a. zur Herstellung steuerbarer Medikamentendepots, kontrastverstärkender Mittel für die NMR-Diagnostik, manipulierbarer Mikrowerkzeuge, als Mittel zur Blockierung von Blutgefäßen und als steuerbare Porogene bei der Membranherstellung verwendet.

Die Erfindung betrifft Polymerträger verschiedener Geometrien und Teilchengrößen, in die eine magnetisierbare und/ oder metallische Substanz oder ein magnetisches- und/oder metallisches Kolloid-enthaltendes Kernpolymer einpolymerisiert sind, die sich in einem hochfrequenten magnetischen Wechselfeld selektiv aufheizen lassen, woraus eine Veränderung der physikalischen Struktur bzw. der Form des polymeren Trägers resultiert, die eine in vivo Applikation dieser Polymerträger ermöglichen. Die Erfindung betrifft ferner die Herstellung und Verwendung der Polymerträger.

Durch Induktion aufheizbare magnetische Polymerpartikel sind in diversen Veröffentlichungen und Patenten beschrieben. So sind in DE-OS 3502998, DE-OS 4201461, DE-OS 4412651 und DE-OS 19800294 induktiv aufheizbare magnetische Polymerpartikel für die Tumortherapie, für die AIDS-Therapie sowie für molekularbiologische Anwendungen beschrieben. Jordan et al., J. Magnet. Mag. Mat., Vol. 225, 118, 2001, sowie Int. J. of Hyperthermia, Vol 9, 51, 1993 verwenden verschieden beschichtete, induktiv aufheizbare magnetische Kolloide zur hyperthermischen Behandlung von Tumoren. In ähnlicher Weise verwenden Mitsumori et al., Int. J. of Hyperthermia, Vol. 10, 785, 1994, und Masuko et al., Biol. Pharm.Bull., Vol. 18, 1802, 1995, induktiv aufheizbare magnetische Partikel bzw. Magnetliposome zur Überwärmung (Hyperthermie) von Tumorzellen.
In den US-Patentschriften 4.735,796 und 4.662.359 werden Magnetpartikel beschrieben, die ebenfalls für die Hyperthermie im Rahmen der Tumortherapie verwendet werden.
Den hier zitierten Mitteln und Verfahren ist gemeinsam, daß die magnetische Induktion ausnahmslos zur Erwärmung der Partikel dient, um Zellen oder biologische Organismen per Überwärmung zu zerstören. Eine Änderung der physikalischen Struktur oder der Form des Polymerträgers mit Hilfe der Induktion kann mit den bekannten Trägern nicht realisiert werden.

Magnetische Mikro- und Nanopartikel vorzugsweise für analytische, diagnostische oder medizinische Zwecke sind aus den Patentschriften PCT/WO 97/04862, PCT/WO 89/11154, PCT/WO 92/22201, PCT/WO 90/07380, PCT/WO 99/62079 sowie den US-Patentschriften 6,020,210, 5,141,740, 4,827,945, 4,647,447, 3,917,538, 4,628,037, 4,827,945, 4,861,705, 4,169,804, 4,115,534, 4,345,588, 4,070,246, 3,970,518, 4,230,685, 4,654,267, 4,452,773, 4,369,226, 4,357,259, 4,861,705, 4,247,406, 4,267,234, 3,652,761, 4,675,173, hier als Referenz beigefügt, allgemein bekannt. Als Polymermatrix kommen bei den vorgenannten Verfahren und Produkten Dextran, Agarose, Dextrin, Albumin, Silicagel, Polystyrol, Gelatine, Polyglutaraldehyd, Agarose-Polyaldehyd-Komposite, Liposome, Polyethylenimin, Polyvinylalkohol, Polyacrolein, Proteine und Polyoxyethylene zum Einsatz, die über gekoppelte Bioliganden bzw. Rezeptoren gemäß dem Affinitätsprinzip Analyten in Form von Antigenen, Antikörpern, Proteinen, Zellen, DNA-Fragmenten, Viren oder Bakterien im Rahmen der biochemisch-medizinischen Analytik und Diagnostik binden können.
Eine Übersicht über weitere mit verschiedenen Polymeren beschichtete Magnetpartikel und deren Anwendung im biomedizinischen Bereich sind in "Scientific and Clinical Applications of Magnetic Carriers", Häfeli et al. Hrsg., Plenum Press, New York, 1997, beschrieben.

Allen vorgenannten Produkten ist gemeinsam, daß sie ihre Funktion ausschließlich aus der komplementären Wechselwirkung eines auf der Matrix gebundenen Bioliganden bzw. -rezeptoren mit der zu analysierenden Substanz ableiten. Ihre Einsatzgebiete beschränken sich somit auf die bekannten Felder der Auftrennung und Analyse von Biomolekülen oder der Markierung bestimmter Zellen unter Ausnutzung des klassischen Affinitätsprinzips.

Die hier als Referenz zitierten magnetischen Polymerträger unterscheiden sich ferner von den erfindungsgemäßen Mitteln dadurch, daß sie, bedingt durch ihre chemische Struktur, nicht thermosensitiv sind, d.h. aufgrund eines äußeren Wärmestimulus ihre physikalische Struktur bzw. geometrische Form nicht in der Lage sind zu ändern. Diese Eigenschaft stellt jedoch die Grundvoraussetzung dar, Polymerträger als manipulierbare oder steuerbare Mikro- oder Nanoträger bzw. -werkzeuge nutzen zu können.

Das meist verwandte polymer mit thermosensitiven Eigenschaften ist Poly-N-Isopropylacrylamid, ein gelartiges Polymer, das bei Temperaturen oberhalb 27°C einem deutlichen Schrumpfungsprozess unterliegt. Diese Schrumpfung ist reversibel, d.h., beim Abfühlen unter 30°C nimmt das Polymer seine ursprüngliche Form praktisch wieder an. Diese spezielle Eigenschaft des Poly-M-Isopropylacrylamids sowie die sich daraus ableitenden interessanten Anwendungen als Medikamentendepot, Biosensor, Zellkultursubstrat, Zelleinkapselungsmatrix, Aktuator oder Ventil sind seit langem bekannt und haben ihren Ausdruck in einer Reihe von Publikationen und Patentschriften gefunden.

Die Polymerisation und das Quellverhalten von N-Isopropylacrylamid oder Copolymerisaten mit beispielsweise Acrylsäure, Methacrylsäure, Polyethylenoxid oder Chitosan sowie die Pfropfcopolymerisation mit Silicongummi oder Polyvinylalkohol werden von Park und Hoffman, J. Biomed. Mat. Res., Vol. 24, 21, 1990, Zhang et al., Langmuir, Vol. 18, 2013, 2002, Lee und Chen, J. Appl. Polymer Sci., Vol. 82, 2487, 2001, Zhu und Napper, Langmuir, Vol. 16, 8543, 2000, Li et al., Radiat. Phys. Chem., Vol. 55, 173, 1999, Zhang und Zhuo, Eur. Polym J., Vol. 36, 2301, 2000, Serizawa et al., Macromolecules, Vol. 35, 10, 2002, Kanazawa et al., Anal. Sci., Vol. 18, 45, 2002, Asano et al., Polym. Gels & Network, Vol. 3, 281, 1995, Sayil und Okay, Polym. Bull., Vol. 45, 175, 2000, Xue et al., Polymer, Vol. 42, 3665, 2001, Maolin et al., Radiat. Phys. Chem., Vol. 57, 481, 2000, sowie Ebara et al., J. Appl. Polymer Sci., Vol. 39, 335, 2001, beschrieben. Entsprechende Nanopartikel oder Mikropartikel aus Poly-N-Isopropylacrylamid als Grundpolymer werden von Gan und Lyon, J. Am. Chem. Soc., Vol. 123, 7517, 2001, Wang et al., J. Am. Chem. Soc., Vol. 123, 11284, 2001, Gilányi et al., Langmuir, Vol. 17, 4764, 2001, West und Halas, Curr. Opinion. Biotechn., Vol. 11, 215, 2000, Matsuoka et al., Polym. Gels & Networks, Vol. 6, 319, 1998 und Jones und Lyon, Macromolecules, Vol. 33, 8301, 2000, beschrieben.

Gegenstand der Arbeit von Kondo und Fukuda, J. Ferment. Bioeng., Vol. 84, 337, 1997, sind magnetische Nanopartikel enthaltende N-Isopropylacrylamid-Copolymere. Das dort beschriebene Verfahren liefert jedoch weder eindeutige Magnetpartikel-Einkapselungen noch sphärische Partikel. Kondo et al., Appl. Microbiol. Biotechn., Vol. 41, 99, 1994, beschreiben magnetische Polystyrolpolymere, die in einer zeitaufwendigen Zweistufenpolymerisation mit Poly-N-Isopropylacrylamid-Methacrylsäure-Copolymeren umhüllt werden. Die Produkte sind ausschließlich zur Separation von Antikörpern geeignet. Eine Wirksubstanz-Applikation in Verbindung mit einer induktiv gesteuerten Freigabe derselben, wie es Gegenstand der vorliegenden Erfindung ist, sind bei beiden Produkten nicht realisierbar.

In den US Patenten 4,832,466, 6,165,389, 6,187,599, 5,898,004, 5,854,078, 6,094,273, 6,097,530, 5,711,884 und 6,014,246 werden thermosensitive optische Systeme in Form von Filtern oder Schalter u.a. unter Verwendung von Poly-N-Isopropylacrylamid Nanopartikeln offenbart. In den US Patentanmeldungen 20020032246, 20020031841, 20010026946 werden u.a. thermosensitive Hydrogele u.a. auf der Basis von N-Isopropylacrylamid für die Separation von Makromolekülen, zur colorimetrischen Detektion von Analyten oder als Sensoren zur Bestimmung chemischer Verbindungen beschrieben.

Thermo- und pH-sensitive Polymergele u.a aus N-Isopropylacrylamid, Hydroxyalkylcellulose, Polyethylenoxid, Polyethylenglycol, Polyvinylalkohol, Dextran, Alkylcellulose, Block-Polymeren aus Polyoxyethylen, Polyoxypropylen, Polyacrylsäure, Ethylendiamin z.B. als Träger für biologischaktive Substanzen gehen aus den US-Patenten 5,674,521, 5,441,732, 5,252,318, 5,599,534, 5,618,800 und 5,840,338 hervor.

Temperatur- und pH-sensitive Polymere aus interpenetrierenden Polymernetzwerken, die u.a. aus Acrylaten, Acrylamiden, Urethanen oder Methacrylaten und Blockcopolymeren aus Polyoxyethylen oder Polyoxypropylen bestehen, sind Gegenstand des US Patentes 5,939,485.

In US-Patent 5,998,588 werden licht-, temperatur- und pH-sensitive interaktive Stimulus-Response Molekülkonjugate aus Poly-N-Isopropylacrylamid für assays oder Separationen beschrieben.
Ebenfalls pH-, licht und temperaturempfindliche Lipid beschichtete Mikropartikel sowie Mikroteilchen und Liposome aus N-substituierten Polyacrylamiden als Medikamentendepot werden in den US-Patenten 5,753,261, 5,226,902 und 5,053,228 offenbart.

Poröse Trägermedien u.a. aus Rayon, Papier, Polyacrylamid-und Agarosebeads als Festphasenträger zur Detektion von Analyten sind im US-Patent 5,013,669 beschrieben.
Auf Acrylat-Trägern immobilisierte Enzyme mit reversiblen Löslichkeiten sind Gegenstand des US-Patentes 4,783,409. Einen thermisch induzierten Phasen-Separations-Immunoassay unter Verwendung von Poly-N-isopropylacrylamid-Copolymeren zur Detektion von Medikamenten, Hormonen, Vitaminen, Proteinen, Metaboliten, Zellen, Viren, Mikroorganismens und Antikörpern wird in US-Patent 4,780,409 offenbart.

Die Synthese und Anwendung von Antikörper-Polymer-Konjugaten ausgehend von N-Hydroxysuccinimid-Acrylaten im Rahmen des Immunassays sowie für analytische Zwecke werden in den US-Patenten 4,752,638 und 4,609,707 veröffentlicht. Rezeptoren, Antikörper, Proteine, Medikamente oder Nukleinsäure enthaltende temperatursensitive Poly-N-Isopropylacrylamid oder Poly-N-Isopropylacrylamid-Copolymere, die in der Lage sind, u.a. Wirkstoffe freizugeben sind Gegenstand des US-Patentes 4,912,032.
Alginat-Beads als orale Medikamentendepot wird in US-Patent 5,451,411 beschrieben.
Bioabbaubare Form-Gedächtnis-Polymere ("shape-memory-polymer"), die aus Hart- und Weichpolymersegmenten bestehen und deren ursprüngliche Form durch Erwärmen auf oberhalb der Glastemperatur wiederhergestellt werden können, sind Gegenstand des US Patentes 6,160,084.

Allen vorgenannten, hier als Referenz aufgeführten Mitteln ist gemeinsam, daß diese, soweit es sich um nicht-magnetische Polymerträger handelt, nur durch direkt von außen zugeführte Wärme zu einer Änderung der physikalischen Struktur oder Form veranlaßt werden können oder, sofern es magnetische Träger sind, weder durch einen äußeren Stimulus noch durch von außen zugeführte Energie strukturell in irgendeiner Weise veränderbar sind. Weiterhin handelt es sich bei den aus dem Stand der Technik bekannten "stimulus-response"-Trägern entweder um unregelmäßige Nanopartikel oder größervolumige Massepolymorinate, die sich nicht als Träger von Wirksubstanzen (Pharmaka), als Kontrastmittel in der NMR-Diagnostik (Magnetresonanztomograph), als Medien für die Molekülauftrennung oder als steuerbare Mikrowerkzeuge für in vivo Applikationen eignen.

Die Aufgabe der vorliegenden Erfindung besteht darin, Polymermatrizes bzw. Polymerträger in nano- oder mikropartikulärer Form sowie anderer Geometrien bereitzustellen, die durch Energiezufuhr in Form einer magnetischen Induktion gezielt so stimuliert werden können, dass aufgrund des daraus resultierenden Erwärmens eine parallele, definierte Änderung der physikalischen Struktur der Polymermatrix herbeigeführt wird.

Unter "Veränderung der physikalischen Struktur" wird dabei definitionsgemäß die Veränderung der geometrischen Form, des Volumens oder der Teilchengröße des Polymerträgers verstanden. Die Volumenänderung kann sich z.B. in einem Schrumpfungs- oder Quellungsprozess mit paralleler Veränderung der Porengröße oder in einer Veränderung der äußeren Form (Geometrie) des Polymeren manifestieren. Veränderung der physikalischen Struktur kann auch die Rückkehr des Polymeren in seine ursprüngliche Form bedeuten, die durch einen Erwärmungs- und Abkühlungsvorgang (Einfrierprozess) zwischenzeitlich verändert wurde ("Form-Gedächtnis-Polymer", "shape-memory-polymer").

Da die Phasenübergangstemperatur (auch: "kritische Lösungstemperatur") dieser Polymeren im Bereich von 27- 38°C liegt, also im Bereich der Körpertemperatur (37°C), können diese Träger bisher nicht in vivo angewendet werden, da bei dieser Temperatur der Schrumpfungsprozess bereits vollzogen ist bzw. die Träger nicht mehr weiter aufgeheizt werden können. Um die Träger auf der Basis von Poly-N-Isopropylacrylamid sowie N-substituierter Acrylamide auch als Träger für Wirksubstanzen therapeutischer, analytischer und diagnostischer Art für eine in vivo Applikation nutzbar zu machen, besteht ein weiterer Gegenstand der Erfindung darin, Wirksubstanzen in die Polymerträger einzukapseln und nach entsprechender in vivo Verabreichung mit Hilfe der magnetischen Induktion gezielt und steuerbar zu applizieren.

Durch die Kombination der magnetfeldinduzierten Erwärmung mit paralleler Änderung der physikalischen Struktur bzw. Trägergeometrie soll ein Eigenschaftsspektrum geschaffen werden, das über das bisheriger Polymerträgersysteme substantiell hinausgeht.

Die Aufgabe der Erfindung wird durch Erwärmung bestimmter Polymerer nach Anspruch 1 mittels magnetischer Induktion, d.h. durch ein von außen angelegtes hochfrequentes magnetisches Wechselfeld, gelöst, indem in die Polymermatrix magnetische und/ oder metallische Substanzen eingekapselt werden, die aus dem Magnetfeld Energie zu absorbieren in der Lage sind und den Polymerträger entsprechend aufheizen können.

Die Aufgabe wird gemäß der Erfindung weiterhin dadurch gelöst, dass spezielle Polymere und Copolymere auf der Basis von Poly-N-Isopropylacrylamid und N-substituierter Acrylamide synthetisiert werden, die auf den Wärmestimulus in Form einer physikalischen Strukturänderung reagieren.

Ausgangsprodukt zur Herstellung der thermonensitiven Polymerträger sind magnetische Kolloide in Form ferromagnetischer, ferrimagnetischer oder superparamagnetischer Nano-oder Mikropartikel, die eine hohe Magnetisierung aufweisen und sich in einem magnetischen Wechselfeld induktiv aufheizen lassen. Die zu diesem Zweck vorzugsweise verwendete Substanz ist Magnetit (Fe₃O₄) oder γ-Fe₂O₃. Die Herstellung solcher Verbindungen ist aus dem Stand der Technik allgemein bekannt: Shinkai et al., Biocatalysis, Vol. 5, 61, 1991, Kondo et al., Appl. Microbiol. Biotechnol., Vol. 41, 99, 1994, Khalafalla und Reimers, IEEE Trans. Magn., Vol. 16, 178, 1980, Lee et al., IEEE Trans. Magn., Vol. 28, 3180, 1992, Buske et al., Colloids & Surfaces, Vol. 12, 195, 1984.

Kolloidale Dispersionen aus Magnetit oder γ-Fe₂O₃ ohne Anwendung irgendwelcher Stabilisatoren wurden von Kang et al., Chem. Mater., Vol. 8, 2209, 1996, veröffentlicht. Ähnliche magnetische Kolloide, die vorwiegend aus Magnetit (Fe₃O₄), Eisenoxid (Fe₂O₃) oder Einenoxybydroxid (FeOOH) bestehen und eine Partikelgröße von 5-100 nm aufweisen und u.a. als Kontrastmittel in der NMR-Diagnostik, als Informationsspeichermedien, Abdicht- oder Dämpfmittel oder zur Zellmarkierung Verwendung finden, gehen aus den folgenden Patentschriften, hier als Referenz beigefügt, hervor: US Patente 5,492,814, 5,221,322, 4,647,447, 4,827,945, 4,329,241, 3,215,572, 3,917,538, DE-OS 350 8000, DE-OS 39 33 210, DE-OS 39 33 210, EP 0 275 285, PCT/IL99/00275, EP 0 586 052.

Weitere substanzen, die die oben beschriebenen Eigenschaften erfüllen und damit für eine Einkapselung in eine Polymermatrix geeignet sind, sind z.B. Ferrite der allgemeinen Formel MOFe₂O₃, worin M ein zweiwertiges Metallion oder ein Gemisch aus zwei zweiwertigen Metallionen oder metallisches Nickel oder Kobalt ist.

Zur Herstellung von Magnetit oder γ-Fe₂O₃ wird durchweg von Eisen(III)- und Eisen(II)-Salzlöslingen mit variierenden molaren Verhältnissen (2:1, 0,5:1 bis 4:1) ausgegangen, die anschließend durch Zugabe von Basen oder durch Hitzezufuhr in entsprechend kolloidale Magnetdispersionen ("Magnetkolloide") überführt werden. Um ein besonders durch die van der Waalssche Kräfte bedingtes Agglomerieren der feinen Magnetpartikel zu verhindern, können oberflächenaktive Stoffe zugesetzt werden, die allgemein unter der Bezeichnung "Tenside", "Emulgatoren" oder "Stabilisatoren" bekannt sind, die ein Absetzen des Kolloids in einer wäßrigen Dispersion praktisch verhindert. Solche stabilisierten kolloidalen Dispersionen sind auch unter der Bezeichnung "Ferrofluide" bekannt ( vgl. Kaiser und Miskolczy, J. Appl. Phys., 413, 1064, 1970). Sie werden heute auch kommerziell angeboten (Ferrofluidics Corp., USA; Advanced Magnetics, USA, Taibo Co, Japan; Liquids Research Ltd., Wales; Schering AG, Deutschland).

Die verwendeten Stabilisatoren sind entweder kationischer-, anionischer oder nicht-ionischer Natur. Geeignete Verbindungen hierfür sind z.B.: Alkylarylpolyethersulfate, Laurylsulfonat, Alkylarylpolyethersulfonate, Phosphatester, Alkoholethersulfate, Zitrate, Ölsäure, Alkylnaphtaleneulfonate, Polystyrolsulfonsäure, Polyacrylsäure oder Petroliumsulfonate als anionische Substanzen, Dodecyltrimethylammoniumchlorid als kationisches Tensid sowie Nonylphenoxypolyglycidol, Polyvinylalkohol, Kerosin, Alkylaryloxypolyethoxyethanole, Nonylphenol oder Polyethylenglykole als nichtionische Substanzen. Die Teilchengrößen der mit Hilfe der vorgenannten Präparationsweisen dargestellten Magnetkolloide hängen, wie allgemein bekannt (s. zitierte Referenzen), von verschiedenen Versuchparametern wie Eisensalzverhältnis, Basenkonzentration, pH-Wert sowie der Temperatur ab.

Die für die erfindungsgemäßen Mittel geeigneten Magnetkolloide weisen durchweg eine Teilchengröße von 5 - 1000 nm, vorzugsweise eine solche von 10 - 500 nm auf. Dadurch ist gewährleistet, daß die Magnetkolloide bei der anschließenden Einkapselung in die Polymermatrix in fein disperser Form vorliegen. Durch gezielte Zudosierung der entsprechenden Mengen des betreffenden Kolloids lassen sich die magnetischen Eigenschaften und analog damit die Aufheizeigenschaften des Polymerträger gezielt steuern.
Die Konzentrationen der Magnetkolloide im Monomeransatz betragen in der Regel 10 bis 30 Vol.%, wobei der Feststoffgehalt der Magnetsubstanz, bezogen auf die Monomerphase, im allgemeinen 5 bis 40 Gew.-%, vorzugsweise 10 bis 30% ausmacht.

Neben den magnetischen Kolloiden können alternativ auch metallische Kolloide in die Polymermatrix eingekapselt werden. Grundsätzlich sind hierfür alle metallischen Werkstoffe in kolloidaler- bzw. fein dispersiver Form geeignet, die sich in einem hochfrequenten Wechselfeld induktiv aufheizen lassen. Da die physiologischen Applikationen bei den erfindungsgemäßen Mitteln einen wesentlichen Aspekt darstellen, werden bevorzugt solche induktiv aufheizbaren Metallkolloide eingesetzt, die physiologisch unbedenklich und/oder chemisch-physikalisch inert sind. Hierzu zählen die Metalle der Gruppe 8 bis 11 (IUPAC Bezeichnung 1986), wobei bevorzugt Gold-, Silber-, Palladium- und Platin-Kolloide oder entsprechende Pulver wegen ihrer Biokompatibilität zum Einsatz gelangen.
Die für die erfindungsgemäßen Mittel eingesetzten Metallkolloide weisen in der Regel eine Partikelgröße zwischen 5 und 300 nm auf. Die Herstellung solcher Kolloide, die seit langem wegen ihrer speziellen Absorptionseigenschaften im sichtbaren Bereich in der Bioanalytik zur Bestimmung von Proteinen und Nukleinsäuren eingesetzt werden, hier vor allem die Goldkolloide, sind aus dem Stand der Technik hinlänglich bekannt: Ackerman et al., J. Histochem. Cytochem., Vol. 31, 433, 1983, Geoghagen et al., J. Histochem. Cytochem., Vol. 24, 1187, 1977, Wang et al., Histochem., Vol. 83, 109, 1985, Birell et al., J. Histochem. Cytochem., Vol. 35, 843, 1987, Köhler et al., Sensors & Actuators B, Vol. 76, 166, 2001, Moeremans et al., Anal. Biochem., Vol. 145, 315, 1985, Englebienne, Analyst, Vol. 123, 1599, 1998, sowie US Patent 6,361,944. Sie werden, wie dem Fachmann auf diesem Gebiet hinlänglich bekannt, durchweg durch Reduktion der entsprechenden Metallsalze oder durch Metallsprühverfahren gewonnen. Metallkolloide oder -Pulver werden darüber hinaus auch vielfältig kommerziell angeboten (Sigma, Aldrich, Fluka).

Für die erfindungsgemäßen Mittel und Verfahren können sowohl die Metallkolloide als auch die entsprechenden Pulver eingesetzt werden, die dem Monomeransatz vor der Polymerisation in der gewünschten Konzentration zugemischt werden. Der Metallanteil in dem Polymeren bzw. in den Partikeln beträgt in der Regel zwischen 5 und 40 Gew.-%.

Nach der Zugabe der Kolloide ist es häufig von Vorteil, die Kolloid-Monomer-Mischung kurzzeitig mit Hilfe eines Ultraschallfingers oder in einem Ultraschallbad zu beschallen, um eine feine Dispersion des Kolloids zu erreichen. Durch die homogene Verteilung des Kolloides ist eine entsprechend bessere Wärmeverteilung in der Polymermatrix möglich, die ihrerseits eine kontinuierliche Freisetzung der eingekapselten Wirksubstanz gewährleistet.

Als thermosensitives Monomer für die Polymermatrix sowie die nano- oder mikropartikulären Träger werden N-Isopropylacrylamid und/oder N-substituierte Acrylamide wie z.B. N-Cyclopropylacrylamid, N-Cyclopropylmethacrylamid, N,N-Diethylacrylamid, N-n-Propylmethacrylamid, N-Isopropylmethacrylamid, N,N-Ethylmethylacrylamid, N-Ethylacrylamid, Propylmethacrylamid sowie H-Acryloylpyrrolidon oder N-acryloylpiperidin verwendet. Bekanntlicherweise (vgl. Referenzen) weist Poly-N-Isopropylacrylamid aufgrund seiner speziellen chemischen Struktur eine Phasenübergangstemperatur zwischen 27 und 38/40°C auf, die in dem Gel oberhalb dieser Temperatur einen deutlichen Schrumpfungsprozess induziert.

Zur Herstellung der erfindungsgemäßen Mittel kommt die Polymerisation in Dispersion bzw. Suspension zum Einsatz bei der die Monomermischung zusammen mit dem betreffenden Kolloid in einer organischen, nicht mit Wasser mischbaren Phase unter Rühren suspendiert und dabei radikalisch polymerisiert wird ("inverse Suspenslonspolymerisation").

Beispiele für solche in der Suspensionspolymerisation benutzten organischen Phasen sind allgemein bekannt: Johansson und Mosbach, Biochim. Biophys. Acta, Vol. 370, 339, 1974. Vornehmlich werden hierfür aromatische Kohlenwasserstoffe wie Toluol oder Benzol, chlorierte Kohlenwasserstoffe, aliphatische Kohlenwasserstoffe oder mineralische bzw. pflanzliche Öle verwendet.

Für die erfindungsgemäßen Mittel und Verfahren haben sich überraschenderweise Kohlenwasserstoffe mit einem polaren Löslichkeitsparameter von 5-10 (cal/cm³)^{1/2} als besonders geeignet erwiesen, wobei hierbei die von K.L. Hoy ("Tables of Solubility Parameters", Union Carbide Corporation, South Charleston, 1969) angegebenen Löslichkeitsparameterwerte für die vorliegende Erfindung zugrunde gelegt werden. Beispiele im sinne der Erfindung sind: 1,2-Dichlorpropan, 1,1,2-Trichlorethan, Trichlorethylen, Bromtrichlomethan, Tetrachlormethan, 1,1,1,2-Tetrachlorethan, Chloroform, 2,3-Dichlorpropanol, 1,2,3-Trichlorpropan.

Neben der Verwendung der betreffenden organischen Lösungsmittel wird die Qualität der Polymerpartikel in bezug auf Dispersität durch die Zugabe bestimmter oberflächenaktiver Substanzen gefördert. Die Erfindung nicht einschränkende Beispiele hierfür sind: Derivate von Polyoxyethylenaddukten, Alkylsulfosuccinate, Polyoxyethylensorbitolester, Polyethylenpropylenoxid- Blockcopolymeren, Alkylphenoxypolyethoxyethanolen, Fettalkoholglycolether-Phosphorsäureester, Sorbitan-Fettsäureester, Sucrosentearat-Palmitatester, Fettalkoholpolyethylenglykolether, Polyglycerinenter, Polyoxyethylenalkohole, Polyoxyethylensorbitan-Fettsäurester und Polyoxyethylensäuren. Um die beim Suspensionsprozess gebildeten Polymertröpfchengröße zu kleinen Werten hin zu verschieben, <1 µm, werden der Dispergierphase üblicherweise 0,3-15 Gew.%, vorzugsweise 0,5 - 5 Gew.-%, eines oder mehrerer Tensid(s)e zugegeben.

Diese Teilchengröße sind vor allem für eine biomedizinische in vivo Applikation geeignet. Partikel mit einer Größe von 20-200 nm finden vorzugsweise Anwendung als Kontrastmittel in der DNA-Diagnostik sowie als Porogene zur Erzeugung einstellbarer Porenweiten in Membranen, solche von 100-500 nm besonders als Medikamentendepot zur gezielten Applikation von Wirksubstanzen z.B. in Form therapeutischer, diagnostischer oder prophylaktischer Agenzien. Diese Partikolgrößen unterstützen die Gewebegängigkeit für in vivo Anwendungen nachhaltig. Ähnliches gilt für die Anwendung der Poly-N-Isopropylacrylamid Partikel als Mittel zur Einstellung definierter Porenweiten in Membranen. Durch Einlagerung von Poly-N-Isopropylacrylamid Nanopartikel in eine beliebige Kunststoffmatrix können Poren erzeugt werden, deren Größe durch induktives Erwärmen und anschließendes Abkühlen zwischen 10 und 80% verkleinert bzw. vergrößert werden können.

Der Dispersionsvorgang wird üblicherweise mit Hilfe eines konventionellen KPG-Rührers oder eines Dispergierwerkzeuges bewerkstelligt. Für Teilchengrößen im Bereich von 10-500 µm genügen herkömmliche Propellerrührer mit Rührgeschwindigkeiten zwischen 600 und 1500 U/Min.. Teilchengrößen von <10 µm sind in der Regel durch Rührgeschwindigkeiten von >1500 U/Min. realisierbar. Dagegen werden für Teilchengrößen <1µm nur Dispergierwerkzeuge mit Rührgeschwindigkeiten von >2000 U/Min. benötigt. Für diese Zwecke kommen vor allem Rührwerkzeuge, die nach den Rotor-Stator-Prinzip arbeiten zur Anwendung. Bei diesen hohen Rührgeschwindigkeiten sind die Experimente vorzugsweise unter Argon- oder Stickstoffatmosphäre oder im Vakuum durchzuführen, um das Einbringen von Luft, das die Dispersionsqualität nachhaltig beeinträchtigt, weitestgehend auszuschalten.

Für die Applikationen besonders im biomedizinischen Bereich hat es sich gezeigt, dass Homopolymere aus N-Isopropylacrylamid allein nicht für eine in vivo Applikation nutzbar sind. Dies hängt mit der Phasenübergangstemperatur der Poly-N-Isopropylacrylamids zusammen, die herstellungsbedingt allgemein zwischen 27 und 38°C liegt. Da diese Temperaturen bereits unterhalb der normalen Körpertemperatur liegen, bedeutet das, das die angestrebten und anwendungsrelevanten Änderungen der physikalischen Struktur im Polymergel bereits stattgefunden haben. Um diese spezifischen Eigenschaften dennoch für die in vivo Applikation nutzen zu können, konnte überraschenaerweise gezeigt werden, dass sich durch eine Copolymerisation des N-Isopropylacrylamid mit Carboxylgruppen-haltigen Comonomeren die Phasenübergangstemperatur zu höheren Werten verschieben läßt, so dass die Funktion der erfindungsgemäßen Mittel in Verbindung mit der induktiven Erwärmung in vollem Umgang genutzt werden kann.

So weisen nano- und mikropartikuläre Acrylsäure- und Methacrylsäure-Copolymerisate, deren Cormonomereagehalt zwischen 0,02 und 3 Mol% liegt, den maximalen Schrumpf oberhalb von 40°C auf. Infolge der inkorporation der teilweise geladenen Carboxylgruppen findet eine grundsätzliche Aufweitung der Polymergele statt, wodurch die hydrophoben Wechselwirkungskräfte, die in der Regel die Schrumpfung des Gels verursachen, bei steigender Temperatur deutlich reduziert werden. Mikropartikuläre Gele mit einer mittleren Teilchengröße von 3,4 µm und einem Acrylsäuregehalt von 1 Mol% weisen bei 38°C nach 4 Minuten unter neutralen pH-Bedingungen eine Reduktion des hydrodynamischen Teilchendurchmessers von 18% gegenüber dem Wert bei Raumtemperatur (20°C) auf, dagegen zeigt derselbe Träger bei >45°C unter sonst gleichen Bedingungen einen Schrumpfgrad von >40%.

Neben dem Schrumpfgrad tragen erhöhte Temperaturen auch dazu bei, die Schrumpfkinetik zu beschleunigen. So beschleunigt sich der Schrumpfprozess bei 45°C im Vergleich zu dem bei 35°C in der Regel um den Faktor 1,5 bis 3.
Das Phänomen der Aufweitung der Polymergele durch Carboxylgruppen-haltige Comonomere kann darüber hinaus auch dazu genutzt werden, die Porengrößen bzw. Porenstruktur der Gele den jeweiligen Einkapselungsaufgaben bestmöglich anzupassen. Höhermolekulare Biomoleküle wie z.B. IgM Antikörper oder das Enzym Galaktosidase, die ein Molmasse >500kDa aufweisen, sind allgemein nicht in der Lage über einen angemessenden Zeitraum (wenige Minuten) aus einem N-Isopropylacrylamid-Homopolymer herauszudiffundieren. Die Porenkanäle sind in diesem Falle dafür zu klein. Durch die beschriebene Copolymerisation mit carboxylgruppen-comonomeren können die Poren jedoch derart aufgeweitet werden, dass auch für solche Biomoleküle eine Diffusion ermöglicht wird. Hierzu reichen Comonomerengehalte von 0,01 bis 2 Mol% in der Regel aus, um die erforderlichen Struktur- und Eigenschaftsmodalitäten herbeizuführen.

Neben der Copolymerisation als die Schrumpfungodynamikunterstützendem Parameter können überraschenderweise auch bestimmte Substanzen, die der Monomermischung vor der Polymerisation zugegeben werden, zu einer Porenerweiterung sowie zu einer Beschleunigung des Schrumpfprozesses beitragen. Stoffe dieser Art, die üblicherweise in einer Konzentration von 2 bis 30 Gew.%, vorzugsweise in einer solchen von 2 bis 20 Gew.-% vorliegen, sind z.B. nanoskalige Silicapartikel, die sich z.B. nach einem Verfahren von Stöber et al., J. Colloid Interface Sci., Vol. 26, 62, 1968, herstellen lassen, desweiteren Polyethylenglylkole oder Polyethylenoxide jeweils mit einem Molmasse zwischen 200 und 5000, ferner Polysaccharide oder abgewandelte Polysaccharide mit einer Molmasse zwischen 500 und 10.000. So verlieren 5-15 vol.% (gezogen auf die Monomerlösung) Polyethylenglykol (Molmasse 400)-enthaltende Poly-N-Isopropylacrylamid Partikel (mittlere Teilchengröße 18 µm) beim Aufheizen auf >45°C innerhalb von 3 Minuten im allgemeinen 5 bis 20% Wasser, während die gleichen Partikel mit einem Polyethylenglykolgehalt von >30% unter sonst gleichen Bedingungen innerhalb von 3 Minuten zwischen 50 und 80% ihres Wassergehaltes verlieren. Mit dem erhöhten Wasserverlust bei steigendem Polyethylenglykolgehalt geht auch eine analoge Steigerung der Schrumpfdynamik einher, die einen direkten Einfluß auf die Freigabekinetik der Wirksubstanzen hat, die in dem Polymerträger eingekapselt sind. So läßt sich durch Zugabe solcher Porogene die Wirksubstanzfreigäbe allgemein um den Faktor 1.5 bis 5 beschleunigen.

Eine weitere Verfahrensweise zu Herstellung nano- und mikropartikulärer Polymerträger besteht darin, N-Isopropylacrylamid auf einen zuvor synthetisierte sphärischen, magnetischen Polymerkern zu pfropfen oder diesen während des Polymerisationsprozesses mit Poly-N-Isopropylacrylamid zu umhüllen bzw. einzukapseln. Dadurch eröffnet sich die Möglichkeit, über die oben beschriebene Suspensionspolymerisation hinaus auch andere Polymerisationsvarianten wie die Fällungspolymersiation und die Emulsionspolymerisation zur Herstellung der erfindungsgemäßen Mittel zu nutzen. Dies eröffnet zusätzlich die Möglichkeit, zu ideal-sphärischen und monodispersen Trägern, die im besonderen die Emulsionspolymerisation liefert, zu gelangen. Mit Hilfe dieser Verfahrens- und Produktkombination lassen sich auf diese Weise neue Produkteigenschaften realisieren, die die Applikationsbreite der neuen Träger signifikant erweitern. Beispielsweise tragen rigide Kernpolymere wie Polystyrol, Polystyrol-Copolymere, Polymethylmethacrylat, Polyglycidylmethacrylat, Silicagel, Polyamid und Polyester dazu bei, die mechanischen Eigenschaften der Polymerträger so zu verbessern, dass diese als Trägermedien für die Säulenchromatographie verwendet werden können.

Durch die induktive Erwärmung des Säulentrennmaterials wechselt das Polymer seine physikalischen Eigenschaften von ursprünglich sehr hydrophil zu relativ hydrophob. Dieser Wechsel hat einen signifikanten Einfluß auf das Separations- und Elutionsverhalten des Trägermedium. Infolge des Phasenüberganges hydrophil-hydrophob werden Proteine wie z.B. Albumin, Fibronectin, Fibrinogen und IgG-Antikörper in der Regel bis zu 60% stärker auf der Trennsäule retendiert als vor dem Phasenübergang. Darüber hinaus kann die Separationscharakteristik des Trennmediums während eines Durchlaufes durch Einschalten des Magnetfeldes signifikant verändert und demzufolge dazu genutzt werden, eine verbesserte Trennqualität bei sonst nur schlecht separierbaren Substanzen zu ermöglichen. Beispiele hierfür sind die Auftrennung von Proteinen, Oligonukleotiden mit gering unterschiedlichen Molmassen sowie die Separation von Steroiden, deren Retentionszeiten oberhalb der Phasenübergangstemperatur im allgemeinen bis zu 70% ansteigen.

Ebenfalls geeignete magnetische Kernpolymere zur Herstellung thermosensitiver Polymerträger sind solche Substrate, die bioabbaubar sind oder sich durch hohe Bikompatibilität auszeichnen. Dadurch kann im besonderen die in vivo Applikation der Trägermatrizes wesentlich verbessert werden. Beispiele für solche Substrate sind Dextran, Gelatine, Polylactide, Polyglykolide, Silicagele, Stärke, Chitosan, Albumin, Polycyanacrylate, Alginat, Polyvinylakohol, Agarose, Polyethylenglykole und Polyethylenoxide. Die Herstellung solcher magnetischen Grundpolymeren geht aus den oben zitierten Referenzen hervor.

Die Einbringung des magnetischen Kernpolymeren in die Matrix geschieht auf zweierlei Weise;
a) durch radikalischen oder strahleninduziertes Aufpfropfen von N-Isopropylacrylamid und b) durch einfaches Einpolymerisieren des Kernpolymeren während des Synthesevorganges.

Die Beschichtung von Polymersubstraten mit Hilfe der strahleninduzierten- sowie der radikalischen Pfropfung in Gegenwart von Cer(IV)-Salzen ist aus dem Stand der Technik allgemein benannt. Sie wird üblicherweise mit wäßrigen 10 bis 30%igen N-Isopropylacrylamid-Lösungen unter Anwendung einer Strahlendosis von 0,2 bis 1 Mrad (2 bis 10 kGy) oder in Gegenwart einer 0,05 bis 0,4 molaren Cer(IV)-Salzlösung durchgeführt. Die entsprechenden Verfahrensweisen gehen aus: DE-OS 4129901, DE-OS 3811042, Müller-Schulte und Horster, Polymer Bull., Vol. 7, 77, 1982, Müller-Schulte und Thomas, Radiat. Phys. Chem., Vol. 35, 93, 1990, Müller-Schulte, Radiat. Phys. Chem., Vol. 42, 891, 1993, Tripathy et al., J. Appl. Polymer Sci., Vol. 81, 3296, 2001, Gupta et al., Biomacromolecules, Vol. 2, 239, 2001, Matsuoka et al., Polym. Gels & Metworks, Vol. 6, 319, 1998, und Li et al., Radiat. Phys. Chem., Vol. 55, 173, 1999, hervor, so dass sie von einem Fachmann auf diesem Gebiet jeder Zeit genutzt werden können.

Analog zur Herstellung der Polymerträger mit Hilfe der inversen Suspensionspolymerisation hat sich auch hier überraschenderweise gezeigt, dass bei der Pfropfung mit N-Isopropylacrylamid die Copfropfung mit Carboxylgruppen-haltigen Monomeren wie Acrylsäure oder Methacrylsäure besonders vorteilhaft ist, da die eingeführten Carboxylgruppen zu einer drastisch erhöhten Schrumfungsdynmik gegenüber dem reinen Pfropfpolymeren Anlaß geben. So weisen N-Isopropylacrylamid gepfropfte, in Wasser nicht quellbare Kernpolymere wie z.B. Polyethylen, Polypropylen, Polyamid, Polyester, Polymethylmethacrylat, Polyglycidylmethacrylat mit einem Pfropfgrad von >40% und einem Acrylsäureaateil von 1-5 Mol% beim Erwärmen von 30°C auf 45°C in der Regel Schrumpfwerte von 50% bis 75% auf, wohingegen die Schrumpfgrade bei Acrlysäuregehalten von <1 Mol% durchweg unter 50% liegen. Bei sonst konstanten N-Isopropylacrylamid- Acrylsäure-Molverhältnissen im Pfropfansatz steigen die Schrumpfgrade mit zunehmendem Gesamtpfropfgrad an.

Die Herstellung der Kernpolymeren geschieht mittels der bekannten Emuleions-, Suspensions- oder Fällungspolymerisation oder der Suspensionavernetzung, die aus folgenden Veröffentlichungen hervorgehen: Li et al., J. Microencapsulation, Vol. 15, 163, 1998, Quellec et al., J. Biomed. Mat. Res. Vol. 42, 45, 1998, Hua et al., J. Mater. Sci. Vol. 36, 731, 2001, Kriwet et al., J. Contr. Release, Vol. 56, 149, 1998, Chu et al., Polym. Bull., vol. 44, 337, 2000, "Methods in Enzymology", Vol. 112, Part A, Widder und Green Hrgs., Academic Press, Inc., Orlando, 1985.
Die Korngrößen der Kernpolymeren lassen sich je nach Anforderung zwischen 50 nm und 1000 nm einstellen.

Ein wesentliches Merkmal der vorliegenden Erfindung besteht darin, die gewünschten Eigenschaften der Polymerträger wie magnetische Eigenschaften, Funktionalität oder Porosität durch die Zusammensetzung der Ausgangsmischung festzulegen. Die Porosität, ein wichtiger Einflußparameter für das Freisetzungsverhalten der eingekapselten Wirksubstanzen, wird in entscheidendem Maße durch die Konzentration des Vernetzeragens im Monomeransatz festgelegt. In der Regel enthält der Monomeransatz zwischen 0,1-10 % Vernetzeranteil (bezogen auf den Gesamtmonomergehalt), vorzugsweise zwischen 0,5% und 5%. Für die Herstellung hochporöser Träger (Porenweite >50 nm) werden in der Regel Vernetzerkonzentrationen <1% verwendet. Als Vernetzer kommen allgemein bi- oder trifunktionelle Monomere in Betracht, die mit der Monomermischung ein statistisches Copolymer bilden. Beispiele für solche di- und trifunktionellen Monomere sind N,N'-Methylenbisacrylamid, Ethylenglykol-dimethacrylat, 1,1,1,-Tris-(hydroxymethyl) -propan-triacrylat, 3-(acryloyloxy)-2-hydroxypropyl-methacrylat, Methacrylsäureallylester und Acrylsäurevinylester.

Zur Initiierung der Polymerisation kommen die allgemein bekannten Radikalbildner zum Einsatz. Durch kombinierte Zugabe von N,N,N',N'-Tetramethyl-ethylendiamin (TEMED) und Ammoniumpersulfat (APS) kann eine signifikante Beschleunigung der Polymerisation erzielt werden. Die Konzentrationen von TEMED und APS (in der Regel 10-40%ige wäßrige Lösungen) liegen, bezogen auf die Monomerphase, im Bereich von 2-8 Vol.% für TEMED und 2-10 Vol.% für APS, wobei generell eine steigende Konzentration an TEMED und APS mit einem proportionalen Anstieg der Polymerisationsgeschwindigkeit einhergeht. Auf diese Weise kann die Polymerisation und damit die Polymerpartikelbildung innerhalb weniger Minuten abgeschlossen werden, die im allgemeinen, wie aus dem Stand der Technik hervorgeht, bis zu 24 Stunden benötigen.

Für die biomedizinische Applikation hat es sich als vorteilhaft erwiesen, X-Isopropylacrylamid mit solchen funktionellen Vinylmonomeren zu copolymerisieren, die über eine kopplungsfähige Gruppe verfügen. Hierfür kommen grundsätzlich solche Comonomere in Frage, die mit X-Isopropylacrylamid polymerisierbar sind und über kopplungsfähige Gruppen in Form von Amino-, Carboxyl-, Epoxy-, Hydroxyl-, Isothiocyanat-, Isocyanat- oder Aldehydfunktionen verfügen. Die Erfindung in keiner Weise einschränkende Beispiele hierfür sind; Acrlysäure, Methacrylsäure, Acrylamid, 2-Bydroxyethyl-methacrylat, 2-Isocyanatoethyl-methacrylat, Acrolein, Hydroxypropyl-methacrylat, 2-Carboxyisopropylacrylamid.

Diese Art der Copolymerisation eröffnet die Möglichkeit, bioaffine Liganden wie Antikörper, Zellrezeptoren, Anti-Zellrezeptor-Antikörper, Nukleinsäure, Oligosaccharide, Lektine und Antigene an die Polymerträger zu koppeln, mit denen sich die thermosensitiven Träger an bestimmte Zielsubstanzen wie Zellen, Biomoleküle, Viren, Bakterien oder Gewebekompartimente dirigieren lassen bzw. sich an diese Zielorgane gemäß dem bekannten Affinitätsprinzip selektiv anlagern. So lassen sich die Polymerträger durch Ankopplung solcher Antikörpern, die gegen die Zelloberflächenstrukturen wie z.B. CD2, CD3, CD4, CD8, CD19, CD14, CD15, CD34 und CD45 ("cluster of differentiation") gerichtet sind, spezifisch an T-Zellen, B-Lymphozyten, Monozyten, Granulozyten, Stammzellen und Leukozyten anlagern.

Mit der zielgerichteten Applikation der erfindungsgemäßen Polymerträger in Verbindung mit einer von außen steuerbaren Strukturveränderung wird überraschenderweise die Möglichkeit eröffnet, neue integrale Wirkkombinationen zu nutzen. Sie bestehen darin, die Polymerpartikel als neuartige kontrastverstärkende Mittel im Rahmen der NMR-Diagnostik und parallel damit als Basis für eine steuerbare Wirkstoffapplikation zu versenden. Aus dem Stand der Technik ist bekannt (DE-OS 3508000, US Patente 5,492,814 und 4,647,447), dass superparamagnetische, ferromagnetische oder paramagnetische Substanzen bei der Bildgebung im Rahmen der NMR-Diagnostik (z.B. Magnetresonanztomografie, MRT) zu einer substantiellen Kontrastvertärkung führen, die ihrerseits eine genauere Diagnose durch bessere Lokalisation und Zuordnung pathologischer Prozesse ermöglicht ( z.B. Erkennung von Tumoren im Frühstadium und Mikrometastasen).

In Verbindung mit der Kopplung bioaffiner Liganden an die Polymermatrix, die eine zielgerichtete Anreicherung der Polymerpartikel in dem zu analysierenden (Zell)Gewebe zuläßt, können die erfindungsgemäßen Mittel Überraschenderweise praktisch parallel sowohl als Träger für therapeutische Wirkstoffe als auch als hochempfindliche diagnostische Nachweismittel herangezogen werden.

Durch Kopplung solcher Antikörper bzw. Antikörper-Fragmente, die gegen ein Tumorzellantigen gerichtet sind, ist zunächst die Voraussetzung geschaffen, die Polymerträger zielgerichtet im Tumorgewebe zu konzentrieren bzw. an die Tumorzellen anzulagern. Beispiele für solche, die Erfindung jedoch nicht einschränkende Tumormarker bzw. -Antigene sind: tumorassozüertes Transplantationsantigen (TATA), Onkofetales Antigen, tumorspezifisches Transplantationsantigen (TSTA), p53-Protein, carzinoembryonales Antigen (CEA), Melanom-Antigene (MAGE-1, MAGE-B2, DAM-6, DAM-10), Mucin (MUC1), humaner Epidermis Rezeptor (HER-2), alpha-Fetoprotein (AFP), Helicose-Antigen (HAGE), humanes Papilloma Virus (HPV-E7), Caspase-8 (CASP-8), CD3, CD10, CD20, CD28, CD30, CD25, CD64, Interleukin-2, Interleukin-9, Mamma-CA-Antigen, Prostata-spezifisches Antigen (PSA), GD2-Antigen, Melanocortin-Rezeptor (MCIR), 138H11-Antigen. Die entsprechenden Antikörper können dabei wahlweise als monoklonale oder polyklonale Antikörper, als Antikörper-Fragmente (Fab, F(ab')₂), als Einzelkettenmoleküle (scFv), als "Diabodies", "Triabodies", "Minibodies" oder bispezifische Antikörper eingesetzt werden.

Zur parallelen Behandlung der Tumore werden die aus der Krebstherapie bekannten Antitumormittel bzw. Cytostatika in die Polymerpartikel eingekapselt. Beispiele hierfür sind: Methotrexat, Cis-Platin, Cyolophosphamid, Chlorambucil, Busulfan, Fluorouracil, Doxorubicin, Ftorafur oder Konjugate dieser Substanzen mit Proteinen, Peptiden, Antikörpern oder Antikörperfragmenten. Konjugate dieser Art sind aus dem Stand der Technik bekannt: "Monoclonal Antibodies and Cancer Therapy, UCLA Symposia on Molecular and Cellular Biology, Reisfeld und Soll, Hrsg., Alan R. Riss, Inc., New York, 1985.

Für die kovalente Anbindung der Bio- bzw. Affinitätsliganden oder Rezeptoren an die Polymerträger werden die bekannten Methoden zur Kopplung bioaktiver Substanzen wie Proteine, Peptide, Oligosaccharide oder Nukleinsäuren an feste Träger genutzt (vgl. "Methods in Enzymology", Mosbach Hrsg, Vol 135, Part B, Academic Press, 1987). Kopplungsagenzien, die hier zum Einsatz gelangen, sind z.B.: Tresylchlorid, Tosylchlorid, Bromcyan, Carbodiimide, Epichlorhydrin, Diisocyanat, Diisothiocyanate, 2-Fluor-1-methyl-pyridiniumtoluol-4-sulfonat, 1,4-Butandiol-diglycidyläther, N-Hydroxysuccinimid, Chlorcarbonat, Isonitril, Hydrazid, Glutaraldehyd, 1,1',-Carbonyl-diimidazol. Darüber hinaus lassen sich die Bioliganden auch über reaktive heterobifunktionelle Verbindungen, die sowohl mit den funktionellen Gruppen der Matrix (Carboxyl-, Hydroxyl-, Sulfhydryl-, Aminogruppen) als auch mit dem Bioliganden eine chemische Bindung eingehen können, koppeln. Beispiele im Sinne der Erfindung sind: Suoccinimidyl-4-(N-maleiimido-methyl)-cyclohexan-1-carboxylat, 4-Succinimidyloxycarbonyl-α-(2-pyxidyldithio)-toluol, Succinimidyl-4-(p-maleimidophenyl)butyrat, N-γ-Maleimidobutyryloxysucoinimidester, 3-(2-Pyridyldithio)propionylhydrazid, Sulfoauccinimidyl-2-(p-azidosalicylamido)-ethyl-1,3'-dithiopropionat. Ein Fachmann auf diesem Gebiet kann diese Kopplungsagenzien jeder Zeit entsprechend den Angaben in "Ullmanns Encyclopädie der Technischen Chemie", 4. Aufl., Bd. 10, oder G.T. Hermanson, "Bioconjugate Techniques", Academic Press, San Diego, 1996 nutzen.

Die magnetischen Eigenschaften der Polymerpartikel werden durch direktes Zumischen eines geeigneten Magnetkolloids oder metallischen Kolloids oder entsprechender Partikel vor der Dispersion zu der Monomerphase erzielt. Durch genaues Zudosieren der Kolloide kann das Aufheizverhalten der Polymerpartikel in gezielter Weise variiert bzw. eingestellt werden. So lassen sich wäßrige Dispersionen, die einen Magnetkolloidanteil von 10 Gew.-% aufweisen, bei einer Magnetfeldamplitude von 30 kA/m und einer Frequenz von 0,8 MHz innerhalb von 30 Sekunden von Raumtemperatur auf ca. 45°C aufheizen. Bei entsprechend höheren Magnetkolloidanteilen steigen die Aufheizwerte analog an. Bei diesen Messungen handelt es sich um die makroskopisch in der Dispersion erfaßten Aufheizraten. Die tatsächlich im Polymerpartikel erzeugte Wärme liegt demzufolge wesentlich höher. Für die Applikation der erfindungsgemäßen Mittel bedeutet das, dass nur eine sehr kurze Induktionsperiode von wenigen Sekunden ausreicht, um einen durch die Erwärmung ausgelösten Stimulus erzeugen zu können. Die Poly-N-Isopropylacrylamid Gele weisen bei Temperaturen >27°C bereits einen deutlichen Schrumpf auf, der, je nach Zusammensetzung des Gels, bis zu 85% in bezug auf das Ursprungsvolumen betragen kann. Der Schrumpfuagsgrad hängt dabei neben dem Comonomerengehalt und Art des Comonomeren, wie oben beschrieben, auch vom Vernetzungsgrad ab. So zeigen Gele mit einem Vernetzungsgrad <1 Mol% in der Regel einen Schrumpfgrad von 60% bis 85%, wohingegen Gele mit einer Vernetzungsgrad von >1 Mol% einen solchen von unter 60% aufweisen.

Um die vorgenannten magnetischen und metallischen Substanzen bzw. Verbindungen auf die für die analytischen, therapeutischen und diagnostischen Anwendungen relevanten Temperaturen aufheizen zu können, bedarf es einer speziellen Auslegung des Magnetfeldes im Hinblick auf die Magnetfeldstärke sowie die Frequenz. Es werden in der Regel stromdurchflossene Spulen benutzt, die von einem Hochfrequenzgenerator gespeist werden. Solche Spulensysteme und Hochfrequenzgeneratoren sind allgemeiner Stand der Technik und werden kommerziell angeboten. Die Abmessungen der Spulen richten sich nach den jeweiligen Probengrößen, sie weisen allgemein einen Durchmesser von 5 bis 30 cm und eine Länge von 5-30 cm auf. Die erforderliche Ausgangsleistung der HF-Generatoren liegt normalerweise zwischen 0,5 und 1,5 kW. Zum Aufheizen der Magnetproben können grundsätzlich zwei Generatoreinstellungen gewählt werden: a) hohe Frequenz im Bereich von 5-20 MHz bei niedriger Magnetfeldstärke von 100-500 A/m oder b) niedrige Frequenz von 0,2-0,8 MHz in Verbindung mit einer hohen Feldstärke von 1 bis 45 kA/m.

Beide Feldparameterkombinationen gewährleisten grundsätzlich eine ausreichende Heizleistung innerhalb eines kurzen Applikationszeitraumes (<1 Min.). Auch für die Bestrahlung größer volumiger Areale, so wie es z.B. bei der Applikation medizinischer Wirksstoffe in bestimmte Körperareale der Fall ist, kann mit größeren Spulengeometrien (30-40 cm Durchmesser) durch entsprechende Erhöhung der Felstärke auf >15kA/m ausreichend Energie zur Aufheizung der Träger zur Verfügung gestellt werden.

Aufgrund der besonderen Produkt- und Verfahreaskombination können die erfindungsgemäßen Polymerträger im besonderen als Matrix zur Einkapselung von Wirksubstanzen sowie als Mittel zur Blockierung von Blutgefäßen herangezogen werden. Unter Ausnutzung der induktiven Aufheizung werden Dosiersysteme für die Verabreichung bzw. Applikation von Wirksubstanzen für den medizinischen Bereich oder die Analytik geschaffen, die sich im besonderen durch ihre kontaktfreie Steuerbarkeit auszeichnen. Unter einer Wirksubstanz wird ein Stoff verstanden, der in irgendeiner Weise eine chemische, biochemische oder physiologische Reaktion auslöst und dabei eine therapeutische, diagnostische und/oder prophylaktische Wirkung erzeugen oder eine analytische Funktion erfüllen kann. Beispiele hierfür sind biologisch aktive Proteine oder Peptide, Enzyme, Antikörper, Antigene, Nukleinsäuren, Glykoproteine, Lektine, Oligosaccharide, Hormone, Lipide, Wachstumsfaktoren, Interleukine, Cytokine, Steroide, Vakzine, Anticoagulantien, zytostatische Agenzien, immuamodulatorische Agenzien oder Antibiotika.

Dazu werden die Wirksubstanzen in die Polymerpartikel eingekapselt. Dies geschieht entweder durch direktes Zumischen der betreffenden Wirksubstanz zu der Monomermischung oder durch Inkubation der Wirksubstanz mit dem zuvor durch eine Wärmebehandlung geschrumpften Polymerträgers. Der infolge des Schrumpfungsprozesses erzeugte Konzentrationsgradient in Richtung Polymergel veranlaßt die Wirksubstanz in das Innere des Gels zu diffundieren.

Bei der ersten Einkapselungsvariante besteht das Problem darin, dass die z.T. recht empfindlichen Wirksubstanzen wie Proteine, Antikörper oder Hormone durch die Polymerisationsbedingungen in irgendeiner Weise geschädigt bzw. inaktiviert werden. Um diesem Problem entgegenzuwirken, hat sich überraschenderweise die Zugabe von Polyalkoholen, Zuckern, Serum Albumin und Gelatine als hilfreich erwiesen, die die Wirksubstanzen gegenüber den Polymerisationseinflüssen nachhaltig zu stabilisieren befähigt sind. Beispiele für solche Substanzen, deren Konzentration im Monameransatz in der Regel zwischen 0,1 und 5 Gew.% beträgt, sind. Inosit, Polyvinylalkohol, Mannit, Sorbit, Aldonit, Erythrit, Sucrose, Glycerin, Xylit, Fructose, Glucose, Galactose oder Maltose.

Die so gewonnenen, mit den betreffenden Wirksubstanzen beladenen Träger können anschließend mit Hilfe der bekannten Verabreichungsmethoden wie Infektion, Implantation, Infiltration, Diffusion, Strömung oder Punktion an die gearünschten physiologischen oder bio-analytischen Wirkorte appliziert werden. Die ortspezifische Applikation der Magnetpartikel kann noch dadurch verstärkt werden, indem die Teilchen mit Hilfe von Elektro- oder starken Permanentmagneten, die von außen an den Reaktionsraum bzw. Wirkort anglegt werden, punktuell an die gewünschten Stellen plaziert werden können. Nachdem die Polymerteilchen ihren Wirkort erreicht haben, können sie durch Anlegen eines hochfrequenten magnetischen Wechselfeldes, das sich außerhalb des eigentlichen Wirk- bzw. Reaktionsbereiches der Polymerträger befindet, auf die entsprechenden Phanenübergangstemperaturen erwärmt werden. Durch die erzeugte Wärme wird ein Schrumpfprozess in dem Polymergel induziert, der eine rasche Freisetzung der eingekapselten Wirkstoffe aus der Matrix auslöst.

Die Zeiten, die die Wirksubstanz benötigt, um aus dem Gel herauszudiffundieren hängen grundsätzlich von der Größe des Gels, der Molmasse des Wirkstoffes, der Temperatur des Gels sowie dem Vernetzungsgrad des Trägers ab. Allgemein gilt, dass geringer vernetzte Gele (0,1 bis 1% Verletzung) sowie Nano- und Mikropartikel eine sehr rasche Diffusion der Wirksubstanz zulassen als höher vernetzte Polymere (>1% Vernetzung) bzw. makroskopische Gele. So können niedermolekulare Hormone wie z.B.Vasopressin, Insulin, Testosteron, Cortison desweiteren Antibiotika, Cytostatika (Molmassee <10 kDa) innerhalb einer Minute zu 80% aus einem 1% vernetzten Nanopartikel, mittlere Teilchengröße 430 nm, beim Erwärmen auf >40°C hinausdiffundieren, während dieselben Wirksubstanzen in einem ca. 5 µm Gelpartikel dafür etwa 5 bis 10 Min. benötigen. Höhermolekulare Wirksoffe wie z.B. Albumin, IgG- Antikörper, Fibrinogen, Lactatdehydrogenase benötigen unter analogen Bedingungen entsprechend längere Zeiten: >10 Minuten. Um die Freisetzungsraten der Wirkstoffe zu verändern, bieten die erfindungsgemäßen Mittel, wie oben gezeigt, eine Reihe einstellbarer bzw. veränderbarer Parameter wie Teilchengröße, Comonomerengehalt, Art des Comonomeren, Erwärmung und/oder Vernetzunggrad, die gewünschten Eigenschaften der Trägermedien so zu verändern, dass eine bestmögliche Anpassung an die jeweiligen Aufgaben möglich ist.

In Verbindung mit der magnetischen Induktion ist erstmals die Basis gegeben, strukturelle Eigenschaftsänderungen von polymeren Trägern so zu nutzen, dass eine kontaktfreisteuerbare Wirksubstanz-Applikation realisiert ist.

Die erfindungsgemäßen Mittel und Verfahren ermöglichen das Quellverhalten der Träger auch in umgekehrter Weise zu nutzen, indem man von einem zuvor per Erwärmung stark geschrumpften Träger ausgeht und diesen anschließend durch einen Abkühlungsvorgang auf unterhalb der Phasenübergangstemperatur wieder in seine gequollene Ursprungsform bzw. - geometrie zurückkehren läßt. Dieses Phänomen läßt sich im Rahmen therapeutischer Antitumor-Maßnahmen anwenden. Eine der fatalen pathologischen Entwicklungen bei der Tumorentwicklung ist die Angiogenese. Hierunter versteht man allgemein eine sich stark ausbreitende Ausbildung von Blutgefäßen im Tumorgewebe. Dieser pathologische Prozess, der bisher vornehmlich medikamentös (oder operativ) behandelt wurde, kann nun überraschenderweise mit Hilfe der erfindungsgemäßen Mittel unterdrückt bzw. stark verzögert werden. Dazu werden Partikel, vorzugsweise mit einer Teilchengröße von 0,3 µm bis 5 µm, die zuvor per Induktion auf Temperaturen >45°C aufgeheizt worden sind und damit ihren maximalen Schrumpfgrad erreicht haben, in das Tumorgewebe eingebracht. Infolge der anschließenden Adaptation an die Körpertemperatur beginnen die Teilchen wieder zu quellen, um schließlich ihren Gleichgewichtsquellzustand nach wenigen Minuten zu erreichen. In diesem gequollenen Zustand üben die Polymerträger eine Embolinatorfunktion aus, d.h., sie sind befähigt, die Blutgefäße zu blockieren und dadurch einer Tumorbildung entgegenzuwirken.

Diese spezielle Funktion auszuüben sind vor allem solche Polymerpartikel befähigt, deren Phasenübergangstemperatur z.B. durch Copolymersiation zu höheren Temperaturen verschoben sind. Besonders befähigt dazu sind solche Träger, die, wie bereits oben erläutert, über Carboxylgruppenhaltige Comonomere verfügen. Hierbei sind vor allem die Träger bevorzugt, die über einen Comonomergehalt von 0,05 bis 1 Mol% verfügen und deren maximale Schrumpftemperatur oberhalb von 40°C liegen. Für die Bekämpfung der Angiogenese sind in der Praxis Teilchen mit einer besonders breiten Größenverteilung geeignet, da hierdurch sämtliche Blutgefäßweiten integral erfaßt werden können.

Die Erfindung wird in den nachfolgenden Beispielen näher erläutert.

### Beispiel 1

10 ml 0,1 M Na-Phosphat-Puffer, pH 7.2, der 15 % aus n-Hexan umkristallisiertes N-Isopropylacrylamid, 5% Acrylamid und 0,6% M,N'-Methylenbisacrylamid enthält, sowie 2,5 ml eines 2,2 mM Fe/ml enthaltenden wäßrigen Magnet-Kolloides (mittlere Teilchengröße 26 nm), das nach einer Vorschrift von Shinkai et al., Biocatalysis, Vol. 5, 61, 1991, hergestellt wurde, werden vermischt und 5 Min. in einem Ultraschallbad (250 W) unter Eiskühlung beschallt. In die Mischung wird sodann für 15 Min. Stickstoff eingeleitet, um überschüssigen Sauerstoff zu entfernen. Der Mischung wird 1 ml einer wäßrigen Lösung, bestehend aus 0,1 mg anti-p53-Antikörper (Roche Molecular Biochemicals), 0,05% Human Serum Albumin, 2% Inosit und 0,5% Gelatine zugegeben. Es wird nochmals 30 Sek. unter Eiskühlung beschallt. Danach wird die wäßrige Phase unter Zuleitung eines Stickstoffstroms mit 2 ml 30%iger Ammoniumpersulfat-Lösung (APS), die 0,5% Igepal 720 enthält, versetzt und anschließend in 150 ml Trichloethylen, die zuvor 20 Min. mit Stickstoff begast wurden und die 1,5% einer aus 80% Span 85 und 20% Tween 20 bestehenden Mischung enthalten, in einem thermostatisierten Dispergiergefäß (Ultra-Turrax LR 1000, IKA Werke) unter Rühren (15.000 U/Min.) bei 4°C suspendiert. Nach 10 Sek. wird 1 ml N,N,N',N'-Tetramethyl-ethylendiamin (TEMED) zugesetzt. Der Suspensionsvorgang wird für 5 Min. unter ständiger Stickstoffzufuhr und Eiskühling fortgesetzt. Man läßt die Dispersion für weitere 20 Min. ohne Rühren bei 10°C weiter polymerisieren. Danach gibt man die Dispersion auf eine mit Stahlwolle dichtgepackte Glassäule (Füllvolumen: ca. 10 ml; Innendurchmesser: 0,5 cm), die von einem 5 cm langen ringförmigen Neodym-Bor-Eisen-Magneten umgeben ist, auf und läßt die Mischung langsam (0,5 ml/Min.) durchtropfen. Nach dem Durchlauf wird zehnmal mit ca. 20 ml 10% Ethanol enthaltendem Na-Phosphat-Puffer, der 2% Inosit und 1.5% Polyvinylakohol (Molmasse,Mw: 5000) enthält, nachgewaschen. Dem schließt sich fünfmaliges Waschen mit dest. Wasser an, gefolgt von dreimaligem Waschen mit 0,05 M Na-Phosphat/1% Inosit-Puffer, pH 7.2. Die magnetische Polymerfraktion auf der Säule wird sodann nach Wegnahme des Magneten mit 5 ml 0,1 M Na-Phosphat-Puffer, pH 7.2, eluiert. Das gewonnene Eluat wird anschließend gefriergetrocknet. Nach Redispersion in 2 ml 0,05 Na-Phosphat/0,1% Human Serum Albumin (HSA)/0,1% Polyethylengylkol (PEG, Mw: 1000)-Puffer, pH 7.5, werden magnetische Polymerpartikel mit einer mittleren Teilchengröße von 170 nm gewonnen. Die gewonnenen Teilchen weisen bei einer Behandlung in einem magnetischen Wechselfeld (Magnetfeld: 30 kA/m; 0,6 MHz, Spulendurchmesser: 5,5 cm, 8 Windungen) innerhalb von zwei Minuten eine Teilchengrößenreduktion von 43% auf.
Die so gewonnenen Teilchen lassen sich als kontrastverstärkende Mittel im Rahmen der NMR-Diagnostik und zur Tumorbehandlung einsetzen.

### Beispiel 2

Kobalt-Ferrit-Nanopartikel (CoFe204) werden nach einer Vorschrift von Sato et al., J. Magn. Magn. Mat., Vol. 65, 252, 1987, aus CoCl₂ und FeCl₃ hergestellt und in Wasser mit Hilfe eines Hochleistungsultraschallfinger (Fa. Dr. Hielscher, 80% Amplitude) in Gegenwart von 0,75% Polyacrylsäure (Mw: 5,500) 30 Sek. dispergiert. 5 ml des 1,9 mM Fe/ml enthaltenden Kolloides mit einer Teilchengröße von 21 nm werden sodann mit 20 ml hochreinem und entgastem Wasser, in dem 15% N-Isopropylacrylamid-, 6% Acrylamid, 1% Acrylsäure, 0,5% Igepal 520 und 0,8% N,N'-Methylenbisacrylamid gelöst sind, vermischt. Die Mischung wird nochmals für eine Min. unter Eiskühlung mit dem Ultraschallfinger und anschließend für 30 Min. im Ultraschallbad beschallt. Nach Zugabe von 2 ml 40% APS wird die Mischung in 300 ml 1,1,1-Trichlorethan, das 6% einer Mischung aus Tween 80 und Span 85 (72% : 28%) enthält, mit Hilfe eines Dispergierwerkzeuges (Ultra-Turrax, IKA Werke, 10.000 U/Min.) unter Eiskühlung und Stickstoffeinleitung dispergiert. Nach 10 Sek. wird 1 ml TEMED zugegeben. Der Dispergiervorgang wird für 5 Min. fortgesetzt. Danach läßt man die Reaktionamischung noch weitere 20 Min. bei 10°C abreagieren. Das Produkt wird anschließend analog Beispiel 1 abgetrennt und gewaschen. Nach der Elution mit 5 ml 0,1 M Na-Phosphat-Puffer, pH 7.4, wird gegen 5 Liter 0,01 M Na-Phosphat-Puffer, pH 7.4, 3 Tage dialysiert. Es werden Magnetpartikel mit einer mittleren Teilchengröße von 245 nm gewonnen. 2 ml der gewonnenen Magnetpartikelfraktion werden auf die Magnettrennsäule aufgegeben (s. Beispiel 1) und dreimal mit 0,01 M HCl Lösung und fünfmal mit hochreinem Wasser gewaschen. Nach Wegnahme des Magneten werden 2 ml 0,1 M 2-Morpholino-ethansulfonsäure (HES)/0,5% PEG (Mw: 1000)-Puffer, pH 4.2, zur Elution der Magnetteilchen auf die Säule aufgegeben. Dem Eluat werden 0,5 ml 0,1 M MES-Puffer, pH 4.2, in dem 0,2 mM N-Cyclohexyl-N'-(2-morpholinoethyl)-carbodiimid-methyl-p-toluolsulfonat gelöst sind, zugegeben. Die Mischung wird für 30 Min. bei Raumtemperatur leicht geschüttelt. Durch nachfolgende Aufgabe auf die mit Stahlwolle gefüllte Trennsäule wird überschüssiges N-Cychohexyl-N'-(2-morpholinoethyl)-carbodiimid-methyl-p-toluolsulfonat abgetrennt und die retendierte Magnetpartikelfraktion fünfmal mit je 15 ml Eiswasser nachgewaschen. Nach Entfernen des Magneten erfolgt die Elution mit 1,5 ml 0,05 M MES-Puffer, pH 5,5. Das Eluat wird mit 0,5 ml desselben MES-Puffers, in dem 1,25 x 10⁻⁴ mM Anti-CD30-Fab-Fragment gelöst sind, versetzt und über einen Zeitraum von 12 Stunden bei 4°C mit dem Antikörper-Fragment gekoppelt. Das Konjugat wird über die Stahlwollegefüllte Säule abgetrennt und zehnmal mit je 10 ml eiskaltem 0,05 M Na-Phosphat/1% inosit/0,1% HSA-Puffer, pH 7.2, nachgewaschen. Dem schließt sich fünfmaliges Waschen mit 0,05 M Glycin-Puffer, pH 10.5, an, gefolgt von zweimaligem Waschen mit dest. Wasser. Nach Wegnahme des Handmagneten wird die Magnetfraktion mit 2 ml 0,1 M Tris/HCl-Puffer, pH 8.5, eluiert. Das Eluat wird mit 3 ml 1 M Glycin enthaltendem Tris-Puffer, pH 8.5, 12 Stunden bei Raumtemperatur inkubiert, um restliches Carbodiimid zu desaktivieren. Die Magnetfraktion wird sodann über die magnetische Säule abgetrennt und zehnmal mit 0,05 M Phosphat-Puffer/0,05% HSA, pH 7.5, nachgewaschen. Nach erfolgter Elution des Magnetkonjugates mit 2 ml 0,05 M Phoophat-Puffer/0,05% HSA, pH 7.5, können die Magnetpartikel nach den bekannten Applikationsverfahren als kontrastverstärkende Mittel im Rahmen der NMR-Diagnostik zur Diagnose von Hodgkin-Lymphomen eingesetzt werden.

### Beispiel 3

7,5 ml 0,1 M Na-Phosphat-Puffer, pH 7.2, in dem 20% N-Isopropylacrylamid, 4 % Acrylamid, 1% N,N'-Methylenbisacrylamid und 2,4% 2-Hydroxyethyl-methacrylat gelöst sind, werden für 20 Min. mit Stickstoff durchspült und anschließend mit 2,5 ml eines Magnetit-Ferrofluides (EMG 507, Fa. Ferro-Tec, USA) versetzt. Die Mischung wird 5 Min. unter Eiskühlung in einem Ultraschallbad beschallt. Es folgt die Zugabe von 2 ml 1% Gelatine und 0,1% HSA enthaltender Insulin-Lösung (Insuman® Basal, 100 I.E./ml). Nach Zugabe von 1,2 ml 35%iger APS-Lösung zu der wäßrigen Phase wird diese in 130 ml Trichloräthylen, die 2,5% Span 60 und 1 % Tween 80 enthält, unter Rühren (1200 U/Min.) und ständiger Eiskühlung sowie einem kontinuierlichen Stickstoffstrom dispergiert. Nach 20 Sek. werden 0,5 ml TEMED zugefügt und die Mischung 8 Min. bei 10°C weitergerührt. Danach läßt man die Mischung für weitere 20 Min. bei 15°C abreagieren. Es erfolgt die Abtrennung der magnetischen Phase und Aufreinigung des retendierten Produktes analog Beispiel 1. Nach Gefrietrocknung und nochmaliger Dispersion in 2 ml Na-Phosphat-Puffer/0,1% HSA/0,5% PEG (Mw:1000), pH 7.2, fallen Magnetpartikel mit einer mittleren Teilchengröße von 23 µm an. Die Exposition der Teilchen in einem Magnetfeld (15 kA/m, 0,6 MHz, Spulendruchmesser: 5,5 cm, 8 Windungen) führt innerhalb von 3 Min. zu einem 55%igen Schrumpf, wobei 58% des ursprünglich zugegebenen Insulins freigesetzt wird. Die Polymerträger lassen sich als Insulin-Depot für die Behandlung von Diabetes verwendet.

### Beispiel 4

1 g 40 Gew.% Magnetit enthaltende Polyvinylalkoholpartikel (mittlere Teilchengröße 25 µm), die gemäß einer Vorschrift von Müller-Schulte und Brunner, J. Chromatogr. A 711 , Vol. 711, 53, 1995, hergestellt wurden, werden mit 4 ml 20%iger wäßriger N-Isopropylacrylamid-Lösung, 0,5 ml N-Vinylpyrrolidon, 3 ml Aceton und 5 ml Ethanol versetzt. Es folgte eine 20 minütige Durchspülung der Mischung mit Argon, der sich eine 45 ständige Bestrahlung mit Gammnastrahlen aus einer Cs137-Quelle (Gammacell 40) (Totaldosis 3,4 kGy) anschließt. Das gepfropfte Material wird anschließend 20 Stunden mit Ethanol extrahiert, gefolgt von einer zehnstündigen Extraktion mit Wasser. Nach Trocknung bis zur Gewichtskonstanz resultiert (bezogen auf das Ausgangspolymer) eine Pfropfausbeute von 67 Gew.%. Induktive Aufheizung auf 40°C ergibt einen Schrumpfungsgrad von 62%. Der so gewonnene Träger kann in der Säulenchromatographie zur Auftrennung von Proteinen genutzt werden.

## Patentansprüche

1. Thermosensitive, magnetische und/oder metallische Kolloide enthaltende Polymere, **dadurch gekennzeichnet, dass** die Polymere aus Poly-N-Isopropylacrylamid oder Poly-N-substituierten Acrylamiden oder Poly-N-substituierten Methacrylamiden oder Copolymeren von Monomeren aus der N-Isopropylacrylamid,
N-substitulerten Acrylamide und N-substituierten Methacrylamide umfassenden Gruppe, oder aus Mischungen der genannten
Polymere oder/und Copolymere bestehen und dass sie durch inverse Suspensionspolymerisation herstellbar sind und eine physikalische Struktur aufweisen, die durch magnetische Induktion veränderbar ist.

2. Thermosensitive, magnetische und/oder metallische Kolloide enthaltende Polymere nach Anspruch 1, **dadurch gekennzeichnet, dass** die Polymere ein oder mehrere Copolymer(e) oder Blockcopolymer(e) enthalten, welche neben dem/den genannten Monomer(en) ein oder mehrere Comonomere, das/die vorzugsweise aus der Gruppe carboxylgruppenhaltiger Monomere wie Acrylsäure, Methacrylsäure, oder aus Acrylaten, Acrylat-Derivaten, Methacrylaten, Methacrylat-Derivaten, Acrolein, Acrylamid, N-substituierten Acrylamiden und Vinylacetat ausgewählt ist/sind, enthalten.

3. Thermosensitive, magnetische und/oder metallische Kolloide enthaltende Polymere nach Anspruch 2, **dadurch gekennzeichnet, dass** die Polymere ein oder mehrere Copolymer(e) oder Blockcopolymer(e) enthalten, welche(s) aus der Gruppe, die Polyacrylsäure, Polyacrolein, Polymethacrylsäure, Polyacrylamid, N -substituierte Polyacrylamide sowie Mischungen derselben umfasst, ausgewählt ist/sind.

4. Thermosensitive, magnetische und/oder metallische Kolloide enthaltende Polymere nach einem der Ansprüche 1 bis 3, **dadurch gekennzeichnet, dass** die Polymere nano- oder mikropartikuläre Teilchen sind.

5. Thermosensitive, magnetische und/oder metallische Kolloide enthaltende Polymere nach einem der Ansprüche 1 bis 4, **dadurch gekennzeichnet, dass** die Änderung der physikalische Struktur durch magnetische Induktion in einem hochfrequenten magnetischen Wechselfeld veränderbar ist

6. Thermosensitive, magnetische und/oder metallische Kolloide enthaltende Polymere nach einem der Ansprüche 1 bis 5, **dadurch gekennzeichet, dass** die Änderung der physikalischen Struktur in einer Änderung der geometrischen Form der Polymeren besteht.

7. Thermosensitive, magnetische und/oder metallische Kolloide enthaltende Polymere nach Anspruch 8, **dadurch gekennzeichet, dass** die Änderung der geometrischen Form in einer Rückkehr zu einer Ursprungsform besteht, die das Polymere vor einer wärmebedingten Formänderung ausweist ("shape-memory-polymer").

8. Thermosensitive, magnetischeund/oder metallische Kolloide enthaltende Polymere nach Anspruch 6 oder 7, **dadurch gekennzeichnet, dass** die Änderung der physikalischen Struktur in einer Vergrößerung oder Verkleinerung der Polymerteilchen besteht.

9. Thermosensitive, magnetische und/oder metallische Kolloide enthaltende Polymere nach einem der Ansprüche 1 bis 8, **dadurch gekennzeichnet, dass** die magnetischen Kolloide aus forromagnetischen, superparamagnetischen, ferrimagnetischen Teilchen, Tief-Temperatur-Ferriten oder aus einem Ferrofluid mit einer Teilchengrösse < 1 µm bestehen.

10. Thermosensitive, magnetische und/oder metallische Kolloide enthaltende Polymere nach Anspruch 9, **dadurch gekennzeichnet, dass** die Tief-Temperatur-Ferrite eine Curie-Temperatur im Bereich von 30°C bis 100°C aufweisen.

11. Thermosensitive, magnetische und/oder metallische Kolloide enthaltende Polymere nach einem der Ansprüche 1 bis 10, **dadurch gekennzeichnet, dass** die metallischen Kolloide aus Elementen der Gruppe 8, 9, 10 oder 11 (Gruppeneinteilung: neuer Vorschlag IUPAC 1986) bestehen.

12. Thermosensitive, magnetische und/oder metallische Kolloide enthaltende Polymere nach einem der Ansprüche 1 bis 11, **dadurch gekennzeichnet, dass** die magnetischen und/oder metallischen Kolloide in Form eines sie umhüllenden Kernpolymeren vorliegen.

13. Thermosensitive, magnetische und/oder metallische Kolloide enthaltende Polymere nach Anspruch 12, **dadurch gekennzeichnet, dass** das Kernpolymer eine Teilchengrösse von 50 bis 1000 nm aufweist.

14. Thermosensitive, magnetische und/oder metallische Kolloide enthaltende Polymere nach einem der Ansprüche 12 oder 13, **dadurch gekennzeichnet, dass** die in dem Kernpolymeren umhüllten magnetischen und/oder metallischen Kolloide in kolloid-disperser Form vorliegen.

15. Thermosensitive Polymere nach einem der Ansprüche 12 bis 14, **dadurch gekennzeichnet, dass** das umhüllende Kernpolymer aus der Gruppe Chitosan, Dextran, Stärke, Polyacrylsäure, Polysacchariden, Silicagel, Siliconderivaten, Cellulose, Proteinen, Albumin, Polyacrylsäure, Agarose, Alginat, Polystyrol, Polyacrylaten, Polymethacrylaten, Polycyanacrylaten, Polymethylmethacrylat, Polyvinylalkohol, Polyamiden, Polyestern, Polyaminosäuren, Hyaluronsäure, Polylactiden, Polyglycoliden, Polyacrolein und Copolymeren derselben stammt.

16. Thermosensitive, magnetische und/oder metallische Kolloide enthaltende Polymere nach einem der Ansprüche 1 bis 15, **dadurch gekennzeichnet, dass** die Polymere 0,1-30 Gew.-% eines Porogens enthalten.

17. Thermosensitive, magnetische und/oder metallische Kolloide enthaltende Polymere nach Anspruch 16, **dadurch gekennzeichnet, dass** das Porogen aus der Gruppe Silicagele, Proteine, Nukleinsäuren, Polyethylenglykole, Polyethylenoxide und Polysaccharide stammt.

18. Thermosensitive, magnetische und/oder metallische Kolloide enthaltende Polymere nach einem der Ansprüche 1 bis 17, **dadurch gekennzeichnet, dass** die Polymere mit einem bi- oder trifunktionellen Vernetzer vernetzt sind.

19. Thermosensitive, magnetische und/oder metallische Kolloide enthaltende Polymere nach Anspruch 18, **dadurch gekennzeichnet, dass** der Vernetzer eine Konzentration, bezogen auf den Gesamtmonomergehalt, von 0,1 % bis 10 % aufweist.

20. Thermosensitive, magnetische und/oder metallische Kolloide enthaltende Polymere nach einem der Ansprüche 1 bis 19, **dadurch gekennzeichnet, dass** die Polymere mit Biomolekülen, die reaktive Gruppen aufweisen, koppeln.

21. Thermosensitive, magnetische und/oder metallische Kolloide enthaltende Polymere nach Anspruch 20, **dadurch gekennzeichnet, dass** die koppelnden Gruppen mit Affinitätsliganden, Peptiden, Proteinen, Antikörpern, Antigenen, Enzymen, Zellrezeptor-Antikörpern, Antikörpern gegen Tumormarker, Antikörperfragmenten, künstlich hergestellten Antikörpern, abgewandelten Antikörpern, Antikörperkenjugaten, Oligosacchariden, Glycoproteinen, Lektinen, Nukleinsäuren, Streptavidin oder Biotin umgesetzt sind.

22. Thermosensitive, magnetische und/oder metallische Kolloide enthaltende Polymere nach einem der Ansprüche 1 bis 21, **dadurch gekennzeichnet, dass** die Polymeren Wirksubstanzen eingekapselt enthalten.

23. Thermosensitive, magnetische und/oder metallische Kolloide enthaltende Polymere nach Anspruch 22, **dadurch gekennzeichnet, dass** die eingekapselten Wirksubstanzen aus der Gruppe Hormone, Cytostatika, Antikörper, Antikörperdrivate, Antikörperfragmente, Cytokine, Immunmodulatoren, Antigene, Proteine, Peptide, Lektine, Glycoproteine, Nukleinsäuren, Antisense-Nukleinsäuren, Oligosaccharide, oder Antibiotika ausgewählt sind.

24. Verfahren zur Herstellung thermosensitive Polymere nach einem der Ansprüche 1 bis 23, **dadurch gekennzeichnet, dass** eine wässrige Monomerlösung, in der magnetische und/oder metallische Kolloide dispergiert sind, nach Zugabe eines multifunktionelien Vernetzer und eines Radikalinitiators in einer mit Wasser nicht mischbaren organischen Phase unter mechanischer Zerkleinerung suspendiert und zu nano- oder mikropartikulären Teilchen radikalisch polymerisiert werden.

25. Verfahren zur Herstellung thermosensltiver Polymere nach einem der Ansprüche 1 bis 23, **dadurch gekennzeichnet, dass** eine wässrige Monomerlösung, in der magnetische und/oder metallische Kolloide dispergiert sind, nach Zugabe eines multifunktionellen Vernetzer in einer mit Wasser nicht mischbaren organischen Phase unter mechanischer Zerkleinerung suspendiert wird und während des Suspensionsvorganges durch Zugabe eines Radikalinitiators zu nano- oder mikropartikulären Teilchen radikalisch polymerisiert wird.

26. Verfahren zur Herstellung thermosensitiver Polymere nach einem der Ansprüche 24 oder 25, **dadurch gekennzeichnet, dass** als Monomer N-isopropylacrylamid-, N-substitulerte Acrylamide, N-substituierte Methacrylamide oder Mischungen derselben verwendet werden.

27. Verfahren zur Herstellung thermosensitiver Polymere nach einem der Ansprüche 24 bis 26, **dadurch gekennzeichnet, dass** der Monomerlösung 0,05 bis 30 Mol % Comonomere zugesetzt werden.

28. Verfahren zur Herstellung thermosensitiver Polymere nach Anspruch 27, **dadurch gekennzeichnet, dass** die Comonomeren Acrylatderivate, Methacrylatderivate, Acrylsäure, Acrolein, Methacrylsäure, Acrylamid, Vinylacetat oder Mischungen derselben sind.

29. Verfahren zur Herstellung thermosensitiver Polymere nach einem der Ansprüche 24 bis 28, **dadurch gekennzeichnet, dass** der Monomerlösung ferromagnotische-, superparamagnetische- oder ferrimagnetische Substanzen oder Nieder-Temperatur-Ferrite oder Ferrofluide mit einer Teilchengrösse < 1 µm zugesetzt werde.

30. Verfahren zur Herstellung thermosensitiver Polymere nach einem der Anspüche 24 bis 29, **dadurch gekennzeichnet, dass** die ferromagnetischen-, superparamagnetischen- oder ferrimagnetischen Substanzen oder Nieder-Temperatur-Ferrite als Kolloide oder In Pulverform vorliegen.

31. Verfahren zur Herstellung thermosensitiver Polymere nach einem der Ansprüche 24 oder 25, **dadurch gekennzeichnet, dass** der Monomerlösung ein nano- oder mikropartikuläres Kernpolymer, in dem magnetische und/oder metallische Kolloide dispersiv eingekapselt sind, zugesetzt wird.

32. Verfahren zur Herstellung thermosensitiver Polymere nach Anspruch 31 **dadurch gekennzeichnet, dass** das Kernpolymer aus Chitosan, Dextran, Stärke, Polyacrylsäure, Polysacchariden, Silicagel, Siliconderivaten, Cellulose, Proteinen, Albumin, Polyacrylsäure, Agarose, Alginat, Polystyrol, Polyacrylaten, Polymethacylaten, Polycyanacrylaten, Polymethylmethacrylat, Polyvinylalkohol, Polyaminosäuren, Hyaluronsäure, Polylactiden, Polyglycoliden, Polyacrolein oder Copolymeren derselben gebildet ist.

33. Verfahren zur Herstellung thermosensitiver Polymere nach einem der Ansprüche 24 und 25, **dadurch gekennzeichnet, dass** die als organische Phase verwendeten Lösungsmittel einen polaren Löslichkeitsparameter von 5-10 (cal/cm³) ^{1/2} aufweisen.

34. Verfahren zur Herstellung thermosensitiver Polymere nach einem der Ansprüche 24 und 25, **dadurch gekennzeichnet, dass** der organischen Phase 0,05 bis 15 Gew.-% einer oder mehrerer oberflächenaktiven Substanz(en) zugesetzt werden.

35. Verfahren zur Herstellung thermosensitiver Polymere nach Anspruch 34, **dadurch gekennzeichnet, dass** die aberflächenaktive Substanz aus der Gruppe Alkylsulfosuccinate, Polyoxyethylenarylether, Polyoxyethylene, Polyoxyethylensorbitanester, Polyokyethylenaddukte. Polyethylenpropylenoxid-Blockcopolymere,
Alkylphenoxypolyethoxyethanolen, Fettalkoholpolyethylenglykolether,
- Polyglycerlnester, Polyoxyethylenalkohole, Polyoxyethylensorbitan-Fettsäurester, Polyoxyethylensäuren und Mischungen derselben stammt.

36. Verfahren zur Herstellung thermosensitiver Polymere nach Anspruch 24, **dadurch gekennzeichnet, dass** die Monomerlösung vor der Dispersion in der organischen Phase 5-120 Sekunden vorpolymertslert wird.

37. Verfahren zur Herstellung thermosensitiver Polymere nach einem der Ansprüche 24 bis 36, **dadurch gekennzeichnet, dass** an die Polymere Affinitätsliganden, Peptide, Proteine, Antikörper, Antigene, Enzyme, Zellrezeptor-Antikörper, Antikörper gegen Tumormarker, Antikörper gegen Tumorantigene, Antikörpertragmente, künstlich hergestellte Antikörper, abgewandelte Antikörper, Antikörperkonjugate, Oligosaccharide, Glycoproteine, Lektine, Nukleinsäuren, Streptavidin oder Biotin gekoppelt werden.

38. Verfahren zur Herstellung thermosensitiver Polymere nach einem der Ansprüche 24 bis 37, **dadurch gekennzeichnet, dass** in die Polymere Wirksubatanzen eingekapselt werden.

39. Verfahren zur Herstellung thermosensitiver Polymere nach Anspruch 38. **dadurch gekennzeichnet, dass** die Wirksubstanzen aus der Gruppe Hormone, Cytostatika, Antikörper, Cytokine, Immunmodulatoren, Antigene, Proteine, Peptide, Lektine, Glycoproteine, Nukleinsäuren, Antisense-Nukleinsäuren, Oligosaccharide, und Antibiotika ausgewählt sind.

40. Verfahren zur Herstellung thermosensitiver Polymere nach einem der Ansprüche 38 oder 39, **dadurch gekennzeichnet, dass** den Wirksubstanzen 0,1 bis 20 Gew.-% mehrwertige Alkohole, Polyvinyalkohol, Gelatine oder Kohlenhydrate zugesetzt werden.

41. Verfahren zur Herstellung thermosensitiver Polymere nach Anspruch 40,
**dadurch gekennzeichnet, dass** die mehrwertigen Alkohole oder Kohlenhydrate aus der Gruppe Inosit, Mannit, Sorbit, Aldonit. Erythrit, Sucrose, Glycerin, Xylit, Fructose, Glucose, Galactose und Maltose stammen.

42. In-vitro Verfahren zur Freisetzung von Wirkstoffen aus wirkstoffhaltigen Partikeln, **dadurch gekennzeichnet, dass** Partikel aus thermosensitiven Polymeren nach einem der Ansprüche 1 bis 23 oder Partikel, die nach einem Verfahren nach einem der Ansprüche 24 bis 41 hergestellt wurden, zwecks magnetischer Induktion in ein magnetisches Wechselfeld gebracht werden, vorzugsweise in ein hochfrequentes magnetisches Wechselfeld.

43. In-vitro Verfahren zum Verändern der physikalischen Struktur von thermosensitiven Polymeren nach einem der Ansprüche 1 bis 23 oder nach einem Verfahren nach einem der Ansprüche 24 bis 41 hergestellten, die magnetische und/oder metalische Kolloide enthalten, oder zum Erwärmen oder Erhitzen solcher Polymeren, **dadurch gekennzeichnet, daß** diese Polymere zwecks magnetischer Induktion in ein magnetisches Wechselfeld gebracht werden, vorzugsweise ein hochfrequentes magnetisches Wechselfeld.

44. Mittel zur Verstärkung des Kontrasts in der NMR-Diagnostik, als Träger für Wirksubstanzen In der medizinischen Therapie und Diagnostik, als steuerbare Träger für Reaktanden, Steuerung von mikrofluiden Prozessen, als Separationsmedium In der Säulenchromatographie, zur Einstellung und Regulierung von Porengrössen in Membranen, zur Blockierung von Blutgefässen, als künstlicher Zellträger, als Separationsmedium für Nukleinsäuren, Zellen, Proteinen, Steroide, Viren oder Bakterion
enthaltend thermosensitiven, magnetische und/oder metallische Kolloide enthaltende Polymere nach einem der Ansprüche 1 bis 23.

## Claims

1. Thermosensitive polymers containing magnetic and/or metallic colloids, **characterized in that** the polymers consist of poly-N-isopropylacrylamide or poly-N-substituted acrylamides or poly-N-substituted methacrylamides or copolymers of monomers from the group comprising N-isopropylacrylamide, N-substituted acrylamides and N-substituted methacrylamides, or of mixtures of said polymers and/or copolymers, and are producable by inverse suspension polymerization, and have a physical structure that is changeable by magnetic induction.

2. Thermosensitive polymers containing magnetic and/or metallic colloids according to claim 1, **characterized in that** the polymers contain one or more copolymer(s) or block copolymer(s) which contain one or more comonomers, in addition to said monomer(s), said comonomer(s) is/are preferably selected from the group of monomers containing carboxyl groups such as acrylic acid, methacrylic acid, or consisting of acrylates, acrylate derivatives, methacrylates, methacrylate derivatives, acrolein, acrylamide, N-substituted acrylamides and vinyl acetate.

3. Thermosensitive polymers containing magnetic and/or metallic colloids according to claim 2, **characterized in that** the polymers contain one or more copolymer(s) or block copolymer(s) which is/are selected from the group comprising polyacrylic acid, polyacrolein, polymethacrylic acid, polyacrylamide, N-substituted polyacrylamides and mixtures thereof.

4. Thermosensitive polymers containing magnetic and/or metallic colloids according to one of claims 1 to 3, **characterized in that** the polymers are nano- or mikroparticulate particles.

5. Thermosensitive polymers containing magnetic and/or metallic colloids according to one of claims 1 to 4, **characterized in that** the change in physical structure is changeable by magnetic induction in a high-frequency magnetic alternating field.

6. Thermosensitive polymers containing magnetic and/or metallic colloids according to one of claims 1 to 5, **characterized in that** the change in the physical structure is a change to the geometric form of the polymers.

7. Thermosensitive polymers containing magnetic and/or metallic colloids according to claim 6, **characterized in that** the change in the geometric form is a return to the original form displayed by the polymers before a change in form caused by heat ("shape-memory-polymer").

8. Thermosensitive polymers containing magnetic and/or metallic colloids according to claim 6 or 7, **characterized in that** the change in the physical structure is an enlargement or reduction in the size of the polymer particles.

9. Thermosensitive polymers containing magnetic and/or metallic colloids according to one of claims 1 to 8, **characterized in that** the magnetic colloids consist of ferromagnetic, superparamagnetic, ferrimagnetic particles, low-temperature-ferrites or a ferrofluid with a particle size of < 1 µm.

10. Thermosensitive polymers containing magnetic and/or metallic colloids according to claim 9, **characterized in that** the low-temperature-ferrites have a Curie temperature in the range of 30 °C. to 100 °C.

11. Thermosensitive polymers containing magnetic and/or metallic colloids according to one of claims 1 to 10, **characterized in that** the metallic colloids consist of elements of the group 8, 9, 10 or 11 (group classification: new suggestion of the 1986 IUPAC definition).

12. Thermosensitive polymers containing magnetic and/or metallic colloids according to one of claims 1 to 11, **characterized in that** the magnetic and/or metallic colloids are present in form of a core polymer encapsulating them.

13. Thermosensitive polymers containing magnetic and/or metallic colloids according to claim 12, **characterized in that** the core polymer has a particle size of 50 to 1000 nm.

14. Thermosensitive polymers containing magnetic and/or metallic colloids according to claim 12 or 13, **characterized in that** the magnetic and/or metallic colloids encapsulated in the core polymer are present in a disperse colloid form.

15. Thermosensitive polymers according to one of claims 12 to 14, **characterized in that** the encapsulating core polymer is from the group of chitosan, dextran, starch, polyacrylic acid, polysaccharides, silica gel, silicone derivatives, cellulose, proteins, albumin, polyacrylic acids, agarose, alginate, polystyrene, polyacrylates, polymethacrylates, polycyanoacrylates, polymethyl methacrylate, polyvinyl alcohol, polyamides, polyesters, polyamino acids, hyaluronic acid, polylactides, polyglycolides, polyacrolein and copolymers of the same.

16. Thermosensitive polymers containing magnetic and/or metallic colloids according to one of claims 1 to 15, **characterized in that** the polymers contain a porogen in an amount of 0.1-30% by weight.

17. Thermosensitive polymers containing magnetic and/or metallic colloids according to claim 16, **characterized in that** the porogen is from the group of silica gels, proteins, nucleic acids, polyethylene glycols, polyethylene oxides and polysaccharides.

18. Thermosensitive polymers containing magnetic and/or metallic colloids according to one of claims 1 to 17, **characterized in that** the polymers are cross-linked with a bi- or tri-functional cross-linking agent.

19. Thermosensitive polymers containing magnetic and/or metallic colloids according to claim 18, **characterized in that** the cross-linking agent has a concentration of 0.1 % to 10% relative to the overall monomer content.

20. Thermosensitive polymers containing magnetic and/or metallic colloids according to one of claims 1 to 19, **characterized in that** the polymers bind to biomolecules which contain reactive groups.

21. Thermosensitive polymers containing magnetic and/or metallic colloids according to claim 20, **characterized in that** the bonding groups are reacted with affinity ligands, peptides, proteins, antibodies, antigens, enzymes, cell receptor antibodies, antibodies against tumor markers, antibody fragments, artificially produced antibodies, modified antibodies, antibody conjugates, oligosaccharides, glycoproteins, lectins, nucleic acids, streptavidin or biotin.

22. Thermosensitive polymers containing magnetic and/or metallic colloids according to one of claims 1 to 21, **characterized in that** the polymers contain active agents encapsulated.

23. Thermosensitive polymers containing magnetic and/or metallic colloids according to claim 22, **characterized in that** the encapsulated active agents are selected from the group of hormones, cytostatic agents, antibodies, antibody derivatives, antibody fragments, cytokines, immunomodulators, antigens, proteins, peptides, lectins, glycoproteins, nucleic acids, antisense-nucleic acids, oligosaccharides or antibiotics.

24. Process for manufacturing thermosensitive polymers according to one of claims 1 to 23, **characterized in that** an aqueous monomer solution, in which magnetic and/of metallic colloids are dispersed, is suspended in an organic phase, that is not miscible with water, through mechanical comminution, after addition of a multifunctional cross-linking agent and a radical initiator, and radically polymerized to nano- or microparticulate particles.

25. Process for manufacturing thermosensitive polymers according to one of claims 1 to 23, **characterized in that** an aqueous monomer solution, in which magnetic and/of metallic colloids are dispersed, is suspended in an organic phase, that is not miscible with water, through mechanical comminution, after addition of a multifunctional cross-linking agent, and radically polymerized to nano- or micropaticulate particles during the suspension process by addition of a radical initiator.

26. Process for manufacturing thermosensitive polymers according to one of claims 24 or 25, **characterized in that** N-isopropylacrylamide, N-substituted acrylamides, N-substituted methacrylamides or mixtures thereof are used as monomer.

27. Process for manufacturing thermosensitive polymers according to one of claims 24 to 26, **characterized in that** 0.05 to 30% by mol co-monomers are added to the monomer solution.

28. Process for manufacturing thermosensitive polymers according to claim 27, **characterized in that** the co-monomers are acrylate derivatives, methacrylate derivatives, acrylic acid, acrolein, methacrylic acid, acrylamide, vinyl acetate or mixtures thereof.

29. Process for manufacturing thermosensitive polymers according to one of claims 24 to 28, **characterized in that** ferromagnetic, superparamagnetic or ferrimagnetic substances, or low-temperature ferrites or ferrofluids with a particle size of < 1 µm are added to the monomer solution.

30. Process for manufacturing thermosensitive polymers according to one of claims 24 to 29, **characterized in that** the ferromagnetic, superparamagnetic or ferrimagnetic substances or low-temperature ferrites are present as colloids or in powder form.

31. Process for manufacturing thermosensitive polymers according to one of claims 24 or 25, **characterized in that** a nano- or microparticulate core polymer, in which magnetic and/or metallic colloids are dispersively encapsulated, is added to the monomer solution.

32. Process for manufacturing thermosensitive polymers according to claim 31, **characterized in that** the core polymer is made of chitosan, dextran, starch, polyacrylic acid, polysaccharides, silica gel, silicone derivatives, cellulose, proteins, albumin, polyacrylic acid, agarose, alginate, polystyrene, polyacrylates, polymethacrylates, polycyanoacrylates, polymethyl methacrylate, polyvinyl alcohol, polyamino acids, hyaluronic acid, polylactides, polyglycolides, polyacrolein or copolymers of the same.

33. Process for manufacturing thermosensitive polymers according to one of claims 24 and 25, **characterized in that** the solvents used as organic phase have a polar solubility parameter of 5-10 (cal/cm³)^{1/2}.

34. Process for manufacturing thermosensitive polymers according to one of claims 24 and 25, **characterized in that** 0.05 to 15% by weight of one or more surfactive substance(s) is/are added to the organic phase.

35. Process for manufacturing thermosensitive polymers according to claim 34, **characterized in that** the surfactive substance is from the group of alkyl sulphosuccinates, polyoxyethylene aryl ethers, polyoxyethylenes, polyoxyethylene sorbitan esters, polyoxyethylene adducts, polyethylene propylene oxide block copolymers, alkylphenoxy polyethoxy ethanols, fatty alcohol polyethylene glycol ethers, polyglycerol esters, polyoxyethylene alcohols, polyoxyethylene sorbitan fatty acid esters, polyoxyethylene acids and mixtures of the same.

36. Process for manufacturing thermosensitive polymers according to claim 24, **characterized in that** the monomer solution is pre-polymerized for 5-120 seconds prior to dispersion in the organic phase.

37. Process for manufacturing thermosensitive polymers according to one of claims 24 to 36, **characterized in that** affinity ligands, peptides, proteins, antibodies, antigens, enzymes, cell receptor antibodies, antibodies against tumor markers, antibodies against tumor antigens, antibody fragments, artificially produced antibodies, modified antibodies, antibody conjugates, oligosaccharides, glycoproteins, lectins, nucleic acid, streptavidin or biotin are bonded to the polymers.

38. Process for manufacturing thermosensitive polymers according to one of claims 24 to 37, **characterized in that** active agents are encapsulated in the polymers.

39. Process for manufacturing thermosensitive polymers according to claim 38, **characterized in that** the active agents are selected from the group of hormones, cytostatic agents, antibodies, cytokines, immunomodulators, antigens, proteins, peptides, lectins, glycoproteins, nucleic acids, antisense-nucleic acids, oligosaccharides and antibiotics.

40. Process for manufacturing thermosensitive polymers according to one of claims 38 or 39, **characterized in that** 0.1 to 20% by weight of polyvalent alcohols, polyvinyl alcohols, gelatins or carbohydrates are added to the active agents.

41. Process for manufacturing thermosensitive polymers according to claim 40, **characterized in that** the polyvalent alcohols or carbohydrates are from the group of inosite, mannite, sorbite, aldonite, erythrite, sucrose, glycerine, xylite, fructose, glucose, galactose and maltose.

42. In-vitro-process for the release of active agents from active agent-containing particles, **characterized in that** particles of thermosensitive polymers according to one of claims 1 to 23 or of particles that were produced by a process according to one of claims 24 to 41 are introduced into a magnetic alternating field for magnetic induction, preferably into a high-frequency magnetic alternating field.

43. In-vitro-process for changing the physical structure of thermosensitive polymers according to one of claims 1 to 23 or of particles that were produced by a process according to one of claims 24 to 41, which contain magnetic and/or metallic colloids, or for warming or heating such polymers, **characterized in that** these polymers are introduced into a magnetic alternating field for magnetic induction, preferably into a high-frequency magnetic alternating field.

44. Means for intensifying the contrast in NMR diagnostics, as carrier for active agents in medical therapy and diagnostics, as controllable carrier for reactants, to control microfluid processes, as separation medium in column chromatography, to adjust and regulate pore sizes in membranes, to block blood vessels, as artificial cell carrier, as separation medium for nucleic acids, cells, proteins, steroids, viruses or bacteria, containing thermosensitive polymers containing magnetic and/or metallic colloids according to one of clams 1 to 23.

## Revendications

1. Polymères thermosensibles contenant des colloïdes magnétiques et/ou métalliques, **caractérisés en ce que** les polymères sont constitués de poly-N-isopropylacrylamide ou d' acrylamides poly-N-substitués ou de méthacrylacrylamides poly-N-substitués ou de copolymères de monomères du groupe comportant le N-isopropylacrylamide, des acrylamides N-substitués et des méthacrylamides N-substitués ou des mélanges desdits polymères et/ou copolymères et **en ce qu'**ils peuvent être fabriqués par polymérisation en suspension inverse et ont une structure physique qui peut être modifiée par induction magnétique.

2. Polymères thermosensibles contenant des colloïdes magnétiques et/ou métalliques selon la revendication 1, **caractérisés en ce que** les polymères incluent un ou plusieurs copolymères ou copolymères à blocs qui incluent en plus dudit ou desdits monomères un ou plusieurs co-monomères qui sont choisis de préférence dans le groupe comportant des monomères à groupe carboxylique, tels que l'acide acrylique et l'acide méthacrylique, ou parmi les acrylates, les dérivés d'acrylates, les méthacrylates, les dérivés de méthacrylates, l'acroléine, l'acrylamide, les acrylamides N-substitués et l'acétate de vinyle.

3. Polymères thermosensibles contenant des colloïdes magnétiques et/ou métalliques selon la revendication 2, **caractérisés en ce que** les polymères incluent un ou plusieurs copolymères ou copolymères à blocs qui sont choisis dans le groupe comportant l'acide polyacrylique, la polyacroléine, l'acide polyméthacrylique, le polyacrylamide, des polyacrylamides N-substitués ainsi que des mélanges de ceux-ci.

4. Polymères thermosensibles contenant des colloïdes magnétiques et/ou métalliques selon l'une des revendications 1 à 3, **caractérisés en ce que** les polymères sont des corpuscules nanoparticulaires ou microparticulaires.

5. Polymères thermosensibles contenant des colloïdes magnétiques et/ou métalliques selon l'une des revendications 1 à 4, **caractérisés en ce que** la modification de la structure physique par induction magnétique peut être effectuée dans un champ magnétique alternatif à haute fréquence.

6. Polymères thermosensibles contenant des colloïdes magnétiques et/ou métalliques selon l'une des revendications 1 à 5, **caractérisés en ce que** la modification de la structure physique consiste à modifier la forme géométrique des polymères.

7. Polymères thermosensibles contenant des colloïdes magnétiques et/ou métalliques selon la revendication 6, **caractérisés en ce que** la modification de la forme géométrique consiste à restaurer une forme d'origine que le polymère avait avant qu'elle ne soit modifiée par la chaleur (« shape-memory-polymer » : polymère à mémoire de forme).

8. Polymères thermosensibles contenant des colloïdes magnétiques et/ou métalliques selon la revendication 6 ou 7, **caractérisés en ce que** la modification de la structure physique consiste à agrandir ou réduire les corpuscules de polymères.

9. Polymères thermosensibles contenant des colloïdes magnétiques et/ou métalliques selon l'une des revendications 1 à 8, **caractérisés en ce que** les colloïdes magnétiques sont constitués de corpuscules ferromagnétiques, superparamagnétiques, ferrimagnétiques, de ferrites basse température ou d'un ferrofluide dont la taille particulaire est inférieure 1 µm.

10. Polymères thermosensibles contenant des colloïdes magnétiques et/ou métalliques selon la revendication 9, **caractérisés en ce que** les ferrites basse température ont une température de Curie dans la gamme de 30°C à 100°C.

11. Polymères thermosensibles contenant des colloïdes magnétiques et/ou métalliques selon l'une des revendications 1 à 10, **caractérisés en ce que** les colloïdes métalliques sont constitués d'éléments du groupe 8, 9, 10 ou 11 (Classement en groupes : nouvelle proposition IUPAC 1986).

12. Polymères thermosensibles contenant des colloïdes magnétiques et/ou métalliques selon l'une des revendications 1 à 11, **caractérisés en ce que** les colloïdes magnétiques et/ou métalliques se présentent sous la forme d'un noyau de polymère qui les enveloppe.

13. Polymères thermosensibles contenant des colloïdes magnétiques et/ou métalliques selon la revendication 12, **caractérisés en ce que** le noyau de polymère a une taille particulaire de 50 à 1000 nm.

14. Polymères thermosensibles contenant des colloïdes magnétiques et/ou métalliques selon l'une des revendications 12 ou 13, **caractérisés en ce que** les colloïdes magnétiques et/ou métalliques enveloppés dans le noyau de polymère se présentent sous la forme d'une dispersion colloïdale.

15. Polymères thermosensibles selon l'une des revendications 12 à 14**, caractérisés en ce que** le noyau de polymère d'enveloppe provient du groupe comportant le chitosane, le dextrane, l'amidon, l'acide polyacrylique, les polysaccharides, le silicagel, les dérivés de silicones, la cellulose, les protéines, l'albumine, l'acide polyacrylique, l'agarose, l'alginate, le polystyrol, les polyacrylates, les polyméthacrylates, les polycyanoacrylates, les polyméthylméthacrylates, le polyvinylalcool, les polyamides, les polyesters, les acides polyaminés, l'acide hyaluronique, les polyactides, les polyglycolides, la polyacroléine, et les copolymères de ceux-ci.

16. Polymères thermosensibles contenant des colloïdes magnétiques et/ou métalliques selon l'une des revendications 1 à 15, **caractérisés en ce que** les polymères contiennent 0,1 à 30% en poids d'un agent porogène.

17. Polymères thermosensibles contenant des colloïdes magnétiques et/ou métalliques selon la revendication 16, **caractérisés en ce que** l'agent porogène est pris dans le groupe comprenant des silicagels, des protéines, des acides nucléiques, des polyéthylène glycols, des oxydes de polyéthylène et des polysaccharides.

18. Polymères thermosensibles contenant des colloïdes magnétiques et/ou métalliques selon l'une des revendications 1 à 17, **caractérisés en ce que** les polymères sont réticulées avec un agent réticulant bi- ou trifonctionnel.

19. Polymères thermosensibles contenant des colloïdes magnétiques et/ou métalliques selon la revendication 18, **caractérisés en ce que** l'agent réticulant est à une concentration allant de 0,1% à 10%, rapportée à la teneur totale en monomères.

20. Polymères thermosensibles contenant des colloïdes magnétiques et/ou métalliques selon l'une des revendications 1 à 19, **caractérisés en ce que** les polymères se couplent à des biomolécules qui comportent des groupes réactifs.

21. Polymères thermosensibles contenant des colloïdes magnétiques et/ou métalliques selon la revendication 20, **caractérisés en ce que** les groupes de couplage sont transformés avec des ligands d'affinité, des peptides, des protéines, des anticorps, des antigènes, des enzymes, des anticorps de récepteurs cellulaires, des anticorps dirigés vers de marqueurs de tumeurs, des fragments d'anticorps, des anticorps artificiels, des anticorps modifiés, des conjugats d'anticorps, des oligosaccharides, des glycroprotéines, des lectines, des acides nucléiques, la streptavidine ou la biotine.

22. Polymères thermosensibles contenant des colloïdes magnétiques et/ou métalliques selon l'une des revendications 1 à 21, **caractérisés en ce que** les polymères contiennent des substances actives encapsulées.

23. Polymères thermosensibles contenant des colloïdes magnétiques et/ou métalliques selon la revendication 22, **caractérisés en ce que** les substances actives encapsulées sont choisies dans le groupe comportant des hormones, des cytostatiques, des anticorps, des dérivés d'anticorps, des fragments d'anticorps, des cytokines, des immunomodulateurs, des antigènes, des protéines, des peptides, des lectines, des glycoprotéines, des acides nucléiques, des acides nucléiques antisens, des oligosaccharides ou des antibiotiques.

24. Procédé de fabrication de polymères thermosensibles selon l'une des revendications 1 à 23, **caractérisé en ce qu'**une solution aqueuse de monomères, dans laquelle sont dispersés des colloïdes magnétiques et/ou métalliques, est suspendue par réduction mécanique en ajoutant un agent réticulant multifonctionnel et un initiateur de radicaux dans une phase organique non miscible dans l'eau et est soumise à une polymérisation radicale pour donner des corpuscules nanoparticulaires ou microparticulaires.

25. Procédé de fabrication de polymères thermosensibles selon l'une des revendications 1 à 23, **caractérisé en ce qu'**une solution aqueuse de monomères, dans laquelle sont dispersés des colloïdes magnétiques et/ou métalliques, est suspendue par réduction mécanique en ajoutant un agent réticulant multifonctionnel dans une phase organique non miscible dans l'eau et est soumise, pendant le processus de suspension, à une polymérisation radicale, en ajoutant un initiateur de radicaux, pour donner des corpuscules nanoparticulaires ou microparticulaires.

26. Procédé de fabrication de polymères thermosensibles selon l'une des revendications 24 ou 25, **caractérisé en ce que** l'on utilise comme monomères des N-isopropylacrylamides, des acrylamides N-substitués, des méthacrylamides N-substitués ou des mélanges de ceux-ci.

27. Procédé de fabrication de polymères thermosensibles selon l'une des revendications 24 à 26, **caractérisé en ce que** l'on ajoute à la solution de monomères 0,05 à 30% molaire de co-monomères.

28. Procédé de fabrication de polymères thermosensibles selon la revendication 27, **caractérisé en ce que** les co-monomères sont des dérivés d'acrylates, des dérivés de méthacrylates, l'acide acrylique, l'acroléine, l'acide méthacrylique, l'acrylamide, l'acétate de vinyle ou des mélanges de ceux-ci.

29. Procédé de fabrication de polymères thermosensibles selon l'une des revendications 24 à 28, **caractérisé en ce que** l'on ajoute à la solution de monomères des substances ferromagnétiques, superparamagnétiques ou ferrimagnétiques, ou des ferrites basse température ou des ferrofluides dont la taille particulaire est inférieure 1 µm.

30. Procédé de fabrication de polymères thermosensibles selon l'une des revendications 24 à 29, **caractérisé en ce que** les substances ferromagnétiques, superparamagnétiques ou ferrimagnétiques ou les ferrites basse température se présentent sous une forme colloïdale ou pulvérulente.

31. Procédé de fabrication de polymères thermosensibles selon l'une des revendications 24 ou 25, **caractérisé en ce que** l'on ajoute à la solution de monomères un noyau de polymère nanoparticulaire ou microparticulaire dans lequel des colloïdes magnétiques et/ou métalliques sont encapsulées de façon dispersive.

32. Procédé de fabrication de polymères thermosensibles selon la revendication 31, **caractérisé en ce que** le noyau de polymère est formé à partir de chitosane, de dextrane, d'amidon, d'acide polyacrylique, de polysaccharides, de silicagel, de dérivés de silicone, de cellulose, de protéines, d'albumine, d'acide polyacrylique, d'agarose, d'alginate, de polystyrol, de polyacrylates, de polyméthacrylates, de polycyanoacrylates, de polyméthylméthacrylate, de polyvinylalcool, d'acides polyaminés, d'acide hyaluronique, de polyactides, de polyglycolides, de polyacroléine, et de copolymères de ceux-ci.

33. Procédé de fabrication de polymères thermosensibles selon l'une des revendications 24 et 25, **caractérisé en ce que** le solvant utilisé comme phase organique a un paramètre de solubilité polaire de 5 à 10 (cal/cm³)^{1/2}.

34. Procédé de fabrication de polymères thermosensibles selon l'une des revendications 24 et 25, **caractérisé en ce que** l'on ajoute à la phase organique 0,05 à 15% en poids d'une ou plusieurs substances tensioactives.

35. Procédé de fabrication de polymères thermosensibles selon la revendication 34, **caractérisé en ce que** la substance tensioactive est prise dans le groupe comportant des alkylsulfosuccinates, l'aryléther de polyoxyéthylène, le polyoxyéthylène, l'ester de sorbitane polyoxyéthylène, des produits d'addition du polyoxyéthylène, des copolymères à blocs de l'oxyde de polypropylène-éthylène, des alkylphénoxypolyéthoxyéthanols, des polyéthylène glycol éther d'alcool gras, des esters de polyglycérine, des polyoxyéthylène-alcools, des esters d'acides gras de polyoxyéthylène sorbitane, des acides polyoxyéthyléniques et des mélanges de ceux-ci.

36. Procédé de fabrication de polymères thermosensibles selon la revendication 24, **caractérisés en ce que** la solution de monomères est prépolymérisée pendant 5 à 120 secondes avant la dispersion dans la phase organique.

37. Procédé de fabrication de polymères thermosensibles selon l'une des revendications 24 à 36, **caractérisé en ce que** l'on couple aux polymères des ligands d'affinité, des peptides, des protéines, des anticorps, des antigènes, des enzymes, des anticorps de récepteurs cellulaires, des anticorps dirigés vers des marqueurs de tumeurs, des anticorps dirigés vers des antigènes de tumeurs, des fragments d'anticorps, des anticorps artificiels, des anticorps modifiés, des conjugats d'anticorps, des oligosaccharides, des glycroprotéines, des lectines, des acides nucléiques, la streptavidine ou la biotine.

38. Procédé de fabrication de polymères thermosensibles selon l'une des revendications 24 à 37, **caractérisé en ce que** des substances actives sont encapsulées dans les polymères.

39. Procédé de fabrication de polymères thermosensibles selon la revendication 38, **caractérisé en ce que** les substances actives sont choisies dans le groupe comportant des hormones, des cytostatiques, des anticorps, des cytokines, des immunomodulateurs, des antigènes, des protéines, des peptides, des lectines, des glycoprotéines, des acides nucléiques, des acides nucléiques antisens, des oligosaccharides et des antibiotiques.

40. Procédé de fabrication de polymères thermosensibles selon l'une des revendications 38 ou 39, **caractérisé en ce que** l'on ajoute aux substances actives 0,1 à 20% en poids d'alcools polyvalents, d'alcool polyvinylique, de gélatine ou d'hydrates de carbone.

41. Procédé de fabrication de polymères thermosensibles selon la revendication 40, **caractérisé en ce que** les alcools polyvalents ou les hydrates de carbone sont pris dans le groupe comportant l'inosite, le mannite, le sorbite, l'aldonite, l'érythrite, le sucrose, la glycérine, le xylite, le fructose, le glucose, le galactose et le maltose.

42. Procédé in vitro de libération de substances actives de particules contenant des substances actives, **caractérisé en ce que** des particules de polymères thermosensibles selon l'une des revendications 1 à 23 ou des particules, qui ont été fabriquées conformément à un procédé selon l'une des revendications 24 à 41, sont amenés dans un champ magnétique alternatif, de préférence dans un champ magnétique alternatif à haute fréquence, dans le but de les soumettre à une induction magnétique.

43. Procédé in vitro de modification de la structure physique de polymères thermosensibles selon l'une des revendications 1 à 23 ou fabriqués conformément à un procédé selon l'une des revendications 24 à 41, lesquels contiennent des colloïdes magnétiques et/ou métalliques, ou de chauffage de ces polymères, **caractérisé en ce que** ces polymères sont amenés dans un champ magnétique alternatif, de préférence un champ magnétique alternatif à haute fréquence, dans le but de les soumettre à une induction magnétique.

44. Moyen d'amplification du contraste dans le diagnostic par résonance magnétique nucléaire (NMR), utilisé comme support pour substances actives en thérapie et diagnostic médicaux, comme support commandé de réactifs, pour commander des processus microfluides, comme fluide de séparation en chromatographie sur colonne, pour régler et réguler la taille des pores de membranes, pour bloquer des vaisseaux sanguins, comme support cellulaire artificiel, comme fluide de séparation d'acides nucléiques, de cellules, de protéines, de stéroïdes, de virus ou de bactéries, lesdits moyens contenant des polymères thermosensibles contenant des colloïdes magnétiques et/ou métalliques selon l'une des revendications 1 à 23.
